(19) Europäisches Patentamt
European Patent Office
Office européen des brevets

(11) Numéro de publication : **0 538 156 A1**

# (12) DEMANDE DE BREVET EUROPEEN

(21) Numéro de dépôt : **92420359.9**

(22) Date de dépôt : **09.10.92**

(51) Int. Cl.$^5$ : **C07D 231/16,** A01N 43/56, C07D 231/14, C07D 231/18, C07D 405/04, C07D 401/06, C07D 401/12, C07D 405/12, C07D 409/12, C07F 9/6506, C07F 7/08, C07D 231/12

(30) Priorité : **09.10.91 FR 9112647**

(43) Date de publication de la demande : **21.04.93 Bulletin 93/16**

(84) Etats contractants désignés : **AT BE CH DE DK ES FR GB GR IE IT LI LU NL PT SE**

(71) Demandeur : **RHONE-POULENC AGROCHIMIE**
**14-20, rue Pierre Baizet**
**F-69009 Lyon (FR)**

(72) Inventeur : **Chene, Alain**
**12, chemin de Montpellas**
**F-69009 Lyon (FR)**
Inventeur : **Peignier, Raymond**
**81bis, chemin de Vassieux**
**F-69300 Caluire (FR)**
Inventeur : **Vors, Jean-Pierre**
**9, chemin de Montpellas**
**F-69009 Lyon (FR)**
Inventeur : **Mortier, Jacques**
**39, rue Louis Bouquet**
**F-69009 Lyon (FR)**
Inventeur : **Cantegril, Richard**
**31, rue de Lattre de Tassigny**
**F-69009 Lyon (FR)**
Inventeur : **Croisat, Denis**
**113, rue Vendôme**
**F-69006 Lyon (FR)**

(74) Mandataire : **Chrétien, François et al**
**RHONE-POULENC AGROCHIMIE- DPI B.P. 9163**
**F-69263 Lyon Cédex 09 (FR)**

(54) **Nouveaux phenylpyrazoles fongicides.**

(57) Ils sont de formule I ou Ib :

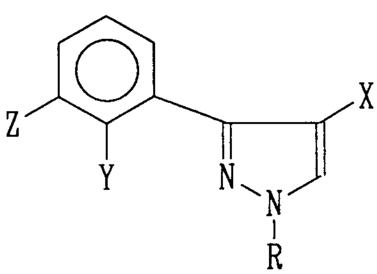

I

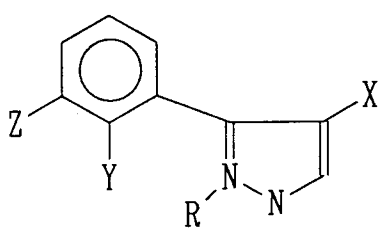

I bis

dans laquelle :

X = H, hal, $NO_2$, CN, SCN, alkyle ($C_1$-$C_4$), alkènyle($C_1$-$C_4$), alkynyle($C_1$-$C_4$), alkoxy ($C_1$-$C_4$), alkylthio ($C_1$-$C_4$), phényle, phénoxy ; mono- ou di-alkyl- ou phenyl-amino ;alkylcarbonyle,carbamoyle, carboxyle, benzoyle ;alkyl-sulfinyle ou -sulfonyle ;

Y, Z = H, hal, OH, $NO_2$, NO, CN, SCN, alkyle ($C_1$-$C_4$), alkènyle($C_1$-$C_4$), alkynyle($C_1$-$C_4$), alkoxy ($C_1$-$C_4$), alkylthio ($C_1$-$C_4$), phényle, phénoxy ; mono- ou di-alkyl- ou phenyl-amino ;alkylcarbonyle,carbamoyle, carboxyle, benzoyle ;alkyl-sulfinyle ou -sulfonyle... ;

Y, Z peuvent aussi former un pont de 1 à 4 atomes, dont au moins un peut être un hétéroatome, éventuellement substitués.

R est H, Hal, $NO_2$, (halo)alkyle, phényle,$S(O)_m$R1,CH2NR3, (CH2)m R4, CH(R5)XR6, CHR7R8, C(X)R10, COXR19,...

EP 0 538 156 A1

Produits utilisables comme fongicides en agriculture.

La présente invention concerne de nouveaux dérivés de la famille des 3-phénylpyrazoles, leurs procédés de préparation, les compositions les contenant et leur utilisation pour la protection des plantes contre les maladies fongiques.

L'invention a plus spécialement pour objet des dérivés 3-phénylpyrazoles, caractérisés en ce qu'ils sont de formules :

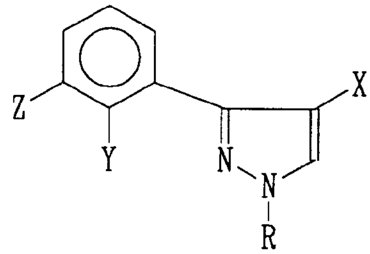

I

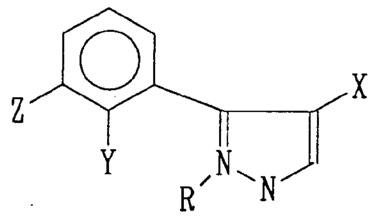

I bis

dans lesquelles:

**X** est:

- un atome d'hydrogène ou d'halogène,
- un groupe nitro, cyano, thiocyanato,
- un groupe alkyle, alkényle, alkynyle, alkoxy ou alkylthio, chacun de ces groupes étant ou non halogéné,
- un phényle ou phénoxy éventuellement substitué,
- un amino substitué ou non par un ou deux alkyles ou phényles,
- un groupe alkylcarbonyle, carbamoyle, carboxyle ou benzoyle
- un groupe alkylsulfinyle ou alkylsulfonyle, étant entendu que la partie alkyle de tous les groupes ci-dessus comprend de 1 à 4 atomes de carbone;

**Y et Z** , identiques ou différents, sont:

- un atome d'hydrogène, mais pas ensemble, ou d'halogène, un groupe hydroxy, nitro, nitroso, cyano, thiocyanato, amino substitué ou non par un ou deux alkyles ou phényles,
- un groupe alkyle, alkoxy ou alkylthio, hydroxyalkyle, alkényle, alkynyle, alkylsulfnyle, alkylsulfonyle, la partie alkyle de ces groupes comprenant de 1 à 4 atomes de carbone et pouvant être halogénée;
- phényle, phényloxy ou phénylthio ou benzyle substitué ou non sur le noyau,
- un groupe formyle, acétyle, alkyl- ou alkoxy (thio)carbonyle, mono ou di alkylamino-carbonyle ou -thio, carboxyle, carboxylate, carbamoyle, la partie alkyle de ces groupes comprenant de 1 à 4 atomes de carbone et pouvant être substituée par au moins un atome d'halogène;
- ou benzoyle, substitué ou non sur le noyau;

Y et Z peuvent également être reliés entre eux par un pont carboné comprenant de 1 à 4 atomes, dont au moins un peut être remplacé par un un atome d'oxygène, de soufre ou d'azote, les carbones de ce pont pouvant en outre être ou non substitués par au moins un atome d'halogène et/ou au moins un groupe alcoyle, alcoxy ou alcoylthio tels que définis ci-dessus, et pouvant chacun encore être reliés, par une double liaison, à un atome d'oxygène;

**R** est:

**-a)** un atome d'hydrogène ou d'halogène, un groupe nitro, amino, hydroxy, cyano, alkyle ou haloalkyle comprenant chacun de 1 à 6 atomes de carbone, un phényle substitué ou non par au moins un atome d'halogène, un groupe nitro ou haloalkyle de 1 à 3 atomes de carbone;

**- b)** un groupe $S(O)_m-R_1$, dans lequel:

- m est un entier de zéro à deux, et
- **$R_1$** est:
  - soit, quand m est égal à zéro:
    - un groupe haloalkyle de 1 à 6 atomes de carbone,
    - un phényle ou 3-pyridyl, chacun pouvant être substitué par au moins un atome d'halogène, un groupe nitro, alkyle, halo alkyle, alkoxy, haloalkoxy, la partie alkyle de ces quatre groupes comprenant de 1 à 4 atomes de carbone;
  - soit, quand m est égal à deux:
    - un groupe alkyle ou alkoxy , chacun comprenant de 1 à 6 atomes de carbone et étant ou non substitué par un ou plusieurs atomes d'halogène ou groupe alkoxy de 1 à 3 atomes de carbone; ou cycloalkyle de 3 à 6 atomes de carbone;

- un groupe alkényle ou alkynyle ou alkénoxy, chacun comprenant de 3 à 6 atomes de carbone;
- un groupe phényle, substitué ou non par 1 à 5 substituants choisis dans le groupe comprenant un atome d'halogène, un groupe nitro, un alkyle, haloalkyle, alkoxy, halohalkoxy comprenant de 1 à 4 atomes de carbone;

**c)** un groupe $CH_2$-$NR_2R_3$, dans lequel:

**- $R_2$** est un groupe:

- alkyle de 1 à 6 atomes de carbone, éventuellement substitué par un substituant choisi dans le groupe comprenant cyano, alkoxy, cycloalkyle de 3 à 7 atomes de carbone alkylcarbonyle, alkoxycarbonyle mono- ou dialkylaminocarbonyle, alkylsulfinyle, alkylsulfonyle, dialkylaminoalkyle, la partie alkyle de ces groupes comprenant de 1 à 6 atomes de carbone;
- alkényle ou alkynyle de 2 à 6 atomes de carbone;
- cycloalkyle de 3 à 7 atomes de carbone;
- phényle ou benzyle éventuellement substitué par un substituant choisi dans le groupe comprenant un atome d'halogène, un groupe cyano, alkyle, alkoxy, haloalkyle ou haloalkoxy de 1 à 9 atomes d'halogène, alkylcarbonyle, alkoxycarbonyle, la partie alkyle de ces groupes comprenant de 1 à 6 atomes de carbone;

**$R_3$** est un groupe:

- Het, Het-alkyle(de 1 à 6 atomes de carbone), Het-alkényle ou Het-alkynyle(chacun de 3 à 6 atomes de carbone), éventuellement substitué par un substituant choisi dans le groupe comprenant un atome d'halogène, un groupe cyano, alkyle, alkoxy, haloalkyle ou haloalkoxy de 1 à 9 atomes d'halogène, alkylcarbonyle, alkoxycarbonyle, la partie alkyle de ces groupes comprenant de 1 à 6 atomes de carbone;

  dans lequel Het est un radical hétérocyclique de 5 à 7 atomes, dont 1 à 3 hétéroatomes (azote,oxygène, soufre), éventuellement substitué par un substituant choisi dans le groupe comprenant un atome d'halogène, un groupe cyano, alkyle, alkoxy, haloalkyle ou haloalkoxy de 1 à 9 atomes d'halogène, alkylcarbonyle, alkoxycarbonyle, la partie alkyle de ces groupes comprenant de 1 à 6 atomes de carbone;
- dialkylaminoalkyle, la partie alkyle de ces groupes comprenant de 1 à 6 atomes de carbone; cycloalkyle ou cycloalkylalkyle(alkyle de 1 à4 atomes de carbone) de 3 à 7 atomes de carbone; ou phénéthyle éventuellement substitué par un substituant choisi dans le groupe comprenant un atome d'halogène, un groupe cyano, alkyle ou alkoxy chacun de 1 à 4 atomes de carbone;

**$R_2$ et $R_3$ peuvent en outre former**, avec l'atome d'azote auquel ils sont reliés, un cycle azoté à 6 atomes, dont 4 sont des atomes de carbone éventuellement substitué et le cinquième est un atome de carbone ou un hétéroatome tel que oxygène, soufre, azote pouvant être substitué par un groupe alkyle, de 1 à 6 atomes de carbone, le cycle azoté pouvant lui même être substitué par un ou deux substituants choisis dans le groupe comprenant cyano, alkylcarbonyle, alkoxycarbonyle, mono- ou dialkylaminocarbonyle, alkylsulfinyle, alkylsulfonyle, la partie alkyle de ces groupes comprenant de 1 à 6 atomes de carbone, phénylsulfinyle, phénylsulfonyle;

**d)** un groupe $(CH_2)_m$- $R_4$, dans lequel:

m est égal à 1 ou 2,

$R_4$ est un groupe cyano, nitro, alkylcarbonyle, phénylcarbonyl, alkoxycarbonyl, mono- ou dialkylaminocarbonyle, P(O),$(alkoxy)_2$, P(O)$(benzyloxy)_2$, P(O)$(phénoxy)_2$, trialkylsilyle, phényle, étant entendu que le radical alkyle de ces groupes comprend de 1 à 4 atomes de carbone et est éventuellement halogéné et que le noyau phényle des radicaux aromatiques peut être substitué par 1 à 5 substituants choisis dans le groupe comprenant un atome d'halogène, un groupe nitro ou un radical alkyle, alkoxy, haloalkyle, haloalkoxy, alkoxycarbonyle, la partie alkyle de chacun de ces radicaux comprenant de 1 à 4 atomes de carbone;

**e)** un groupe CH(R5)-X-R6, dans lequel:

- $R_5$ est un atome d'hydrogène ou un alkyle de1 à 4 atomes de carbone,
- X est un atome d'oxygène ou un groupe $S(O)_n$, dans lequel:
- n est un entier égal à zéro ou deux,
- $R_5$ est:
  - alkyle de 1 à 4 atomes de carbone éventuellement substitué par un substituant choisi dans le groupe comprenant un atome d'halogène, un groupe cyano, alkoxy , phénoxy, benzyloxy, trialkylsilyl( la partie alkyle de chacun de ces radicaux comprenant de 1 à 4 atomes de carbone et les noyaux phényle peuvent être substitués par 1 à 5 substituants choisis dans le groupe comprenant un atome d'halogène, un groupe nitro ou un radical alkyle, alkoxy, haloalkyle, haloalkoxy,( la partie alkyle de chacun de ces radicaux comprenant de 1 à 4 atomes de carbone);
- alkényle ou alkynyle de 3 à 6 atomes de carbone;
- phényle ou benzyle pouvent être substitués par 1 à 5 substituants choisis dans le groupe comprenant un atome d'halogène, un groupe nitro ou un radical alkyle, alkoxy, haloalkyle, haloalkoxy,(1 à 4 atomes de carbone);

**f)** un groupe $CHR_7R_8$, dans lequel:
- $R_7$ est un atome d'hydrogène, un groupe haloalkyle ou alkoxy, chacun de 1 à 4 atomes de carbone,
- $R_8$ est un atome d'halogène, un groupe hydroxy, alkoxy ou O-C(O)-$R_9$ avec
- $R_9$ étant un atome d'hydrogène, un groupe alkyle, haloalkyle, alkényle 1 à 4 atomes de carbone, tetrahydrofuryle, tetrahydropyranyle ou alkoxycarbonyle,
  la partie alkyle de chacun de ces radicaux comprenant de1 à 6 atomes de carbone;

**g)** un groupe C(X)-$R_{10}$, dans lequel:
- X est un atome d'oxygène ou de soufre,
- $R_{10}$ est:
  - un atome d'hydrogène ou d'halogène, un groupe alkyle de1 à 6 atomes de carbone, éventuellement substitué par un substituant choisi dans le groupe comprenant un atome d'halogène, un groupe cyano, nitro, alkylcarbonyle, alkoxycarbonyle, mono- ou dialkylaminocarbonyle, la partie alkyle de chacun de ces radicaux comprenant de 1 à 4 atomes de carbone; un groupe cycloalkyle de3 à 6 atomes de carbone;
  - un groupe alkényle ou alkynyle, de 3 à 6 atomes de carbone, éventuellement substitué par un phényle pouvant être substitué par 1 à 5 substituants choisis dans le groupe comprenant un atome d'halogène, un groupe nitro ou un radical alkyle, alkoxy, haloalkyle, haloalkoxy, la partie alkyle de chacun de ces radicaux comprenant de1 à 4 atomes de carbone;
  - un groupe phényle, benzyle, 2-pyridyle, 3-pyridyle, 4-pyridyle, les noyaux pouvant être substitués par 1 à 5 substituants choisis dans le groupe comprenant un atome d'halogène, un groupe nitro ou cyano, ou un radical alkyle, alkoxy, haloalkyle, haloalkoxy, alkylcarbonyle ou alkoxycarbonyle, la partie alkyle de chacun de ces radicaux comprenant de1 à 4 atomes de carbone;
- un groupe CH($R_{11}$)-X-$R_{12}$, dans lequel:
  - $R_{11}$ est un atome d'hydrogène ou un alkyle de 1 à 4 atomes de carbone,
  - X est un atome d'oxygène ou le groupe $S(O)_p$, avec p égal à zéro ou 2;
  - R12 est un alkyle de 1 à 4 atomes de carbone, éventuellement substitué par un atome d'halogène ou un alkoxy de 1 à 4 atomes de carbone; un groupe alkényle ou alkynyle, de 3 à 6 atomes de carbone; un groupe phényle ou benzyle pouvant être substitués par 1 à 5 substituants choisis dans le groupe comprenant un atome d'halogène, un groupe nitro ou un radical alkyle, alkoxy, haloalkyle, haloalkoxy,(1 à 4 atomes de carbone);
- un groupe CH($R_{11}$)-N$R_{13}R_{14}$, dans lequel $R_{13}$ et$R_{14}$, identiques ou différents, sont chacun:
  - un alkyle de 1 à 4 atomes de carbone, éventuellement substitué par un groupe cyano, alkylcarbonyle, alkoxycarbonyle ou dialkylaminocarbonyle,atome d'halogène ou un alkoxy de 1 à 4 atomes de carbone
  - un groupe phényle ou benzyle pouvent être substitué par 1 à 5 substituants choisis dans le groupe comprenant un atome d'halogène, un groupe nitro, cyano ou un radical alkyle, alkoxy, haloalkyle, haloalkoxy, alkoxycarbonyle, la partie alkyle de chacun de ces radicaux comprenant de1 à 4 atomes de carbone;
- un groupe $CHR_{11}$-$R_{15}$, dans lequel:
  - $R_{11}$ est comme défini ci-dessus et
  - $R_{15}$ est un groupe hétérocyclique $NC_4R_{16}R_{17}T$, dans lequel $R_{16}$ et-$R_{17}$, identiques ou différents, sont un atome d'hydrogène, un groupe alkyle ou alkoxycarbonyle chacun de 1 à 3 atomes de carbone, et T est un atome d'oxygène ou de soufre, un groupe carbonyle ou N-$R_{18}$, dans lequel $R_{18}$ est un atome d'hydrogène, un groupe alkyle, formyle,alkylcarbonyl ou alkoxycarbonyle, la partie alkyle de chacun de ces radicaux comprenant de1 à 4 atomes de carbone;

**h)** un groupe-C(O)-X-$R_{19}$, dans lequel:
- X est un atome d'oxygène ou de soufre,
- $R_{19}$ est:
  - un groupe alkyle de1 à 6 atomes de carbone, éventuellement substitué par un substituant choisi dans le groupe comprenant un atome d'halogène, un groupe cyano; un groupe cycloalkyle de 3 à 6 atomes de carbone, éventuellement substitué par un alkyle de 1 à 3 atomes de carbone; trialkylsilyle, phénylsulfonyle éventuellement substitué par au moins un atome d'halogène ou un groupe alkyle; alkoxycarbonyle, dialkylaminocarbonyle; la partie alkyle de chacun des radicaux

précédents comprenant de 1 à 4 atomes de carbone; un groupe cycloalkyle de3 à 6 atomes de carbone;

- un groupe cycloalkyle de3 à 6 atomes de carbone, éventuellement substitué par un alkyle de1 à 3 atomes de carbone,
- un groupe alkényle ou alkynyle, de 2 à 6 atomes de carbone, éventuellement substitué par un phényle pouvant être substitué par 1 à 5 substituants choisis dans le groupe comprenant un atome d'halogène, un groupe nitro ou un radical alkyle;
- un groupe phényle, benzyle, 2-pyridyle, 3-pyridyle, 4-pyridyle, les noyaux pouvant être substitués par 1 à 5 substituants choisis dans le groupe comprenant un atome d'halogène, un groupe nitro ou cyano, ou un radical alkyle, alkoxy, haloalkyle, haloalkoxy, alkylcarbonyle ou alkoxycarbonyle, alkylthio, alkylsulfinyle, alkylsulfonyle, la partie alkyle de chacun de ces radicaux comprenant de1 à 4 atomes de carbone;
- un groupe phénylalkyle ou un groupe hétérocyclylalkyle, dans lequel la partie alkyle comprend de1 à 4 atomes de carbone, et la partie héterocyclyle peut être 2-pyridyle, 3-pyridyle, 4-pyridyle, 2-furyl, 3-furyl, 2-thiényl, 3-thiényl, les noyaux pouvant être substitués par 1 à 5 substituants choisis dans le groupe comprenant un atome d'halogène, un groupe nitro, ou un radical alkyle, alkoxy, haloalkyle, haloalkoxy, alkylcarbonyle ou alkoxycarbonyle, alkylthio, alkylsulfinyle, alkylsulfonyle.

**i)** un groupe C(X)-$NR_{20}R_{21}$, dans lequel:

- X est un atome d'oxygène ou de soufre,
- $R_{20}$ et $R_{21}$, sont chacun:
  - un atome d'hydrogène ou un groupe alkyle de1 à 4 atomes de carbone, éventuellement substitué par un substituant choisi dans le groupe comprenant un atome d'halogène, un groupe cyano, alkylcarbonyle, alkoxycarbonyle, dialkylaminocarbonyle, la partie alkyle de chacun des radicaux précedents comprenant de 1 à 4 atomes de carbone;
  - un groupe cycloalkyle de 3 à 6 atomes de carbone, éventuellement substitué par un alkyle de1 à 3 atomes de carbone,
  - un groupe alkényle ou alkynyle, de 3 à 6 atomes de carbone,
  - un groupe phényle, benzyle, dont les noyaux peuvent être substitués par 1 à 5 substituants choisis dans le groupe comprenant un atome d'halogène, un groupe nitro ou cyano, ou un radical alkyle, alkoxy, haloalkyle, haloalkoxy, alkylcarbonyle ou alkoxycarbonyle, alkylthio, alkylsulfinyle, alkylsulfonyle, la partie alkyle de chacun de ces radicaux comprenant de1 à 4 atomes de carbone;

$R_{20}$ et $R_{21}$ peuvent en outre former, avec l'atome d'azote auquel ils sont reliés un cycleà 6 atomes, dont 4 sont des atomes de carbone éventuellement substitué et le cinquième est un atome de carbone ou un hétéroatome tel que oxygène, soufre,;

**j)** un groupe $SiR_{22}R_{23}R_{24}$, dans lequel $R_{22,}$ $R_{23}$ et $R_{24}$ identiques ou différents, sont chacun un groupe alkyle, de1 à 4 atomes de carbone, ou un groupe phényle ou benzyle,

**k)** un groupe $P(X)R_{25}R_{26}$, dans lequel :

- X est un atome d'oxygène ou de soufre,
- $R_{25}$ et $R_{26}$ identiques ou différents, sont chacun un groupe alkyle ou alkoxy, de1 à 4 atomes de carbone, ou un groupe phényle, phénoxy, benzyle ou benzoxy.

De préférence, dans la formule, X est un atome de chlore ou de brome.

D'autres dérivés préférés sont tels que, dans les formules I et I bis, Y et/ou Z sont un atome d'hydrogène ou de chlore.

D'autres dérivés préférés sont tels que ,dans les formules I et I bis, Y et Z forment ensemble un pont comprenant 3 ou 4 atomes.

D'autres dérivés préférés sont tels que ,dans les formules I et I bis, Y et Z forment un pont méthylènedioxi éventuellement halogéné.

D'autres dérivés préférés sont tels que ,dans les formules I et I bis, R est un alkyl(1 à 3 atomes de carbone)carbonyle, alkyl(1 à 3 atomes de carbone)oxycarbonyle, phénylcarbonyle ou phényloxycarbonyle.

**Les dérivés selon l'invention peuvent être préparés** à l'aide de divers procédés en soi connus notamment dans les compilations "Comprehensive Heterocyclic Chemistry",A.R.Katritzky et C.W.Rees 1984, Vol.5, pages 239 à 241 et 263, Pergamon Press ; "Advances in Heterocyclic Chemistry",A.N.Kost et I.I.Grandberg, 1966, Vol.6,pages 391 à396, Academic Press et "The chemistry of heterocyclic compounds", L.C.Behr, R.Fusco et C.H.Jardoe,1967 J.Wiley & sons.

Un **premier procédé** consiste à faire réagir un 3-phénylpyrazole de formule II, dans laquelle Y et Z ont la même signification que dans la formule I, avec un **agent d'halogénation.**

Comme agent d'halogénation on peut citer , comme agent de chloration, le chlore, de préférence en milieu aqueux comme dans l'eau, ou organique tel que l'acide acétique ou le tétrachlorure de carbone, ou encore

l'acide hypochloreux, l'acide chlorhydrique en présence d'eau oxygénée dasn l'acide acétique, ou encore le chlorure de sulfuryle ou un N-chloroimide tel que le N-chlorosuccinimide dans un solvant chloré tel que le dichlorométhane, ou encore le pentachlorure de phosphore.

La chloration peut être effectuée avec le chlore en milieu solvant organique , de préférence un acide carboxylique inférieur, à une température de 16 à 30°C, et de préférence à température ambiante, les réactifs étant dans un rapport molaire sensiblement stoechiométrique.La chloration peut également être effectuée avec le N-chlorosuccinimide en milieu solvant organique, de préférence un solvant chloré tel que le dichloroéthane, le 1,2 dichloroéthane à une température de 0°C à 80°C, et de préférence de 20°C à 50°C, les réactifs étant dans un rapport molaire sensiblement stoechiométrique.

Comme agent de bromation, on peut citer le brome, de préférence dans un solvant, aqueux tel que l'eau, en milieu acide par exemple nitrique ou acétique,en présence ou non d'une base tel que l'acétate de sodium, ou dans un solvant organique comme par exemple le chloroforme,ou encore le perbromure de pyridinium.

La bromation peut être effectuée par exemple, avec du brome en milieu solvant organique tel qu'un acide carboxylique inférieur, à une température de 16°C à de préférence à la température ambiante.

Comme agent de iodation, on peut utiliser l'iode en présence d'acide hypoiodeux ou en présence d'une base comme un hydroxyde alcalin ou un sel basique tel que l'acétate de sodium, ou en présence d'un sel de nickel(II). On peut encore utiliser l'iode sur le sel d'argent (I) du pyrazole de formule I. On peut également utiliser le N-iodosuccinimide, comme indiqué ci-dessus pour le N-chlorosuccinimide.

La fluoration peut s'effectuer à partir de dérivés de formule V, dans laquelle Y et Z ont la même signification que dans la formule I, par préparation du dérivé tétrafluoroborate de diazonium dérivé de groupe amine en 4 puis irradiation de ce composé.

Un second procédé en soi connu de préparation des dérivés de formule I selon l'invention, dans laquelle consiste à faire réagir un 4-formyl-3- phénylpyrazole de formule IV avec du brome dans l'acide acétique pour donner le 4-bromo-3-phénylpyrazole.

Les composés de formule II, peuvent être préparés ,de manière en soi connue, par réaction avec l'hydrazine d'un dérivé de formule III, dans laquelle X est un atome d'hydrogène et W est un radical hydroxyle ou un atome de chlore et Y et Z ont les mêmes significations que dans la formule I.

On peut également préparer de manière en soi connue les dérivés de formule II à partir de dérivés de formule III, dans laquelle X est un atome d'hydrogène et W un groupe dialcoylamino, Y et Z étant définis comme précedemment, par réaction avec de l'hydrate d'hydrazine, à une température de 10°C à 150°C,de préférence de 60°C à 120°C, avantageusement en milieu solvant organique, de préférence un acide carboxylique inférieur ou dans un alcoolen présence d'un catalyseur acide organique ou minéral, le rapport molaire des 2 réactifs étant sensiblement stoechiométrique.

Les dérivés de formule III, dans laquelle X est un atome d'hydrogène et W un groupe dialcoylamino, Y et Z étant définis comme précedemment,peuvent être obtenus ,de manière en soi connue, par réaction d'acétophénones de formule IV, dans lesquelles Y et Z sont définis comme précedemment, avec des acétals d'amide, des aminals d'esters ou des orthoaminals, de préférence en l'absence de solvant organique avec des dialcoyl(de préférence diméthyl ou diéthyl) acétals du N,N-diméthylformamide, à une température de 20°C à 130°C et de préférence de 70°C à 130°C.

Les exemples suivants sont donnés à titre indicatif pour illustrer la préparation et l'activité fongicide des dérivés selon l'invention. La structure de ces derniers a été confrmée par analyse RMN.

EXEMPLE 1 :

On dissout, à température ambiante et sous agitation, 25g(0, 162 mole) de 2'-choloroacétophénone dans 60 ml de N,N-diméthylformamide diméthylacétal. L'agitation est maintenue et le mélange réactionnel est chauffé pendant 4h30 à 80°C. Le milieu est concentré à sec, sous pression réduite. Le résidu est repris avec 150 ml de chlorure de méthylène. La solution organique résultante est lavée à l'eau, séchée sur $MgSO_4$ ,puis concentrée. Le résidu est chromatographié sur colonne de silice(éluant : heptane/acétate d'éthyle 50/50). On obtient 27,0g(0, 129 mole) de 1-(2-chlorophényl) 3-diméthylamino 2-propène-1-one(composé 1) (rendement 80%), sous forme de miel. dont la structure est confirmée par RMN.

EXEMPLE 2 :En opérant comme à l'exemple 1 mais en utilisant des réactifs appropriés, on a obtenu les dérivés de formule III rassemblés dans le tableau A suivant:

| COMPOSE N° | Y | Z | Rdt(%) | F(°C) ou analyse |
|---|---|---|---|---|
| 2 | F | H | 66 | RMN |
| 3 | H | Cl | 63 | 72 |
| 4 | Cl | Cl | 87 | 89 |
| 5 | $OCF_2$ | O | 76 | 84 |
| 6 | $SCH_3$ | H | 95 | RMN |
| 7 | $CH_3$ | H | 69 | RMN |
| 8 | H | $CH_3$ | 73 | 45 |
| 27 | $nC_3H_7$ | Cl | 100 | RMN |
| 28 | $CH_3$ | $CF_3$ | 100 | RMN |
| 29 | H | $OCF_3$ | 95 | RMN |
| 30 | $OCH_3$ | Cl | 97 | RMN |
| 31 | Cl | Br | 91 | 114 |
| 32 | H | $CF_3$ | 79 | 62 |
| 33 | $C_2H_5$ | Cl | 80 | 94 |
| 34 | F | Br | 75 | 54 |
| 35 | H | CN | 93 | 118 |
| 36 | $O(CH_2)_2$ | O | 65 | RMN |
| 37 | $SOCH_3$ | Cl | 93 | 102 |
| 38 | Cl | F | 88 | 72 |
| 39 | $CH_3$ | Cl | 84 | 76 |
| 40 | F | Cl | 83 | 64 |
| 41 | $OCH_2$ | O | 57 | 118 |
| 42 | F | $CF_3$ | 72 | 59 |
| 43 | $OCH_3$ | $OCH_3$ | 83 | RMN |
| 44 | F | F | 79 | 62 |
| 45 | $CH_3$ | H | 69 | RMN |
| 46 | H | F | 84 | 73 |
| 47 | naphtyl | naphtyl | 100 | huile |
| 48 | $NO_2$ | Cl | 73 | 170 |
| 49 | $C_6H_5CH_2O$ | H | 68 | 75 |

| | | | | |
|---|---|---|---|---|
| 50 | $CH_3$ | $CH_3$ | 65 | solide |
| 51 | Cl | $NO_2$ | 73 | 116 |
| 52 | H | $NO_2$ | 67 | 105 |
| 53 | $NO_2$ | H | 80 | 132 |
| 54 | H | Br | 89 | huile |
| 55 | $(CH_3)_3Si$ $CH_2$ | Cl | 83 | miel |
| 56 | Br | H | 99 | huile |
| 57 | $CF_3$ | H | 98 | huile |
| 58 | $NO_2$ | $CH_3$ | 95 | 161 |
| 59 | $C_2H_5O$ | Cl | 100 | miel |

EXEMPLE 3 :

On ajoute, lentement et à température ambiante, 7g(0,0540 mole) d'hydrate d'hydrazine à une solution de 10,5g(0,050 mole) de 1-(2-chlorophényl) 3-diméthylamino 2-propène 1-one ,préparée à l'exemple 1, dans 60 ml d'éthanol. Le mélange réactionnel est agité pendant 5 heures à température ambiante puis laissé au repos pendant 12 heures, avant d'être concentré à sec. Le résidu est recristallisé dans un mélange heptane/acétate d'éthyle. On obtient 6,9g (0,0386 mole) de 3-(2'-cholorophényl) 1H-pyrazole, qui fond à 93°C (rendement 77%)(composé 60).

**EXEMPLE 4:**

En opérant comme à l'exemple 3 mais en utilisant des réactifs appropriés, on a obtenu les dérivés de formule II, rassemblés dans le tableau B suivant :

| COMPOSE N° | Y | Z | Rdt(%) | F(°C) ou analyse |
|---|---|---|---|---|
| 10 | F | H | 73 | 51 |
| 11 | H | Cl | 96 | 84 |
| 12 | Cl | Cl | 90 | 121 |
| 13 | $OCF_2$ | O | 80 | 130 |
| 14 | $SCH_3$ | H | 53 | 90 |
| 15 | $CH_3$ | H | 92 | RMN |
| 16 | H | $CH_3$ | 61 | RMN |
| 61 | $nC_3H_7$ | Cl | 41 | RMN |
| 62 | H | $OCF_3$ | 87 | 98 |
| 63 | $OCH_3$ | Cl | 94 | 100 |
| 64 | Cl | Br | 50 | 160 |
| 65 | H | $CF_3$ | 86 | 67 |
| 66 | $C_2H_5$ | Cl | 63 | 77 |
| 67 | F | Br | 75 | 121 |
| 68 | H | CN | 90 | 85 |
| 69 | $CF_3$ | H | 67 | 73 |
| 70 | $SOCH_3$ | Cl | 86 | 168 |
| 71 | Cl | F | 100 | 120 |
| 72 | $CH_3$ | Cl | 87 | 74 |
| 73 | F | Cl | 81 | 103 |
| 74 | $OCH_2$ | O | 91 | 50 |
| 75 | F | $CF_3$ | 90 | 86 |
| 76 | $OCH_3$ | $OCH_3$ | 86 | 90 |
| 77 | F | F | 86 | 90 |
| 78 | $CH_3$ | H | 93 | RMN |
| 79 | H | F | 76 | 77 |
| 80 | naphty 1 | naphty 1 | 81 | 119 |
| 81 | $NO_2$ | Cl | 46 | 173 |
| 82 | $C_6H_5CH_2O$ | H | 83 | 62 |

| | | | | |
|---|---|---|---|---|
| 83 | $CH_3$ | $CH_3$ | 66 | 79 |
| 84 | Cl | $NO_2$ | 93 | 117 |
| 85 | H | $NO_2$ | 70 | 126 |
| 86 | $NO_2$ | H | 95 | 80 |
| 87 | H | Br | 89 | 106 |
| 88 | $(CH_3)_3Si$ $CH_2$ | Cl | 56 | miel |
| 89 | Br | H | 72 | 134 |
| 90 | $CF_3$ | H | 67 | 72 |
| 91 | $NO_2$ | $CH_3$ | 81 | 137 |
| 92 | $NO_2$ | $SC_6H_5$ | 8 | 164 |
| 93 | $C_2H_5O$ | Cl | | 96 |

EXEMPLE 5 :

On dissout, à température et sous agitation, 3,5g de 3-(2-chlorophényl) 1H-pyrazole, préparé comme à l'exemple 9, dans 20ml d'acide acétique. Une solution de 3,76g (0,0235n mole) de brome dans 20ml d'acide acétique est ensuite coulée, goutte à goutte, dans le milieu réactionnel. L'agitation est maintenue pendant 1 heure à température ambiante puis l'acide acétique et l'excès de brome sont chassés sous pression réduite. Le résidu est repris au chlorure de méthylène et cette solution organique est lavée à l'eau bicarbonatée, puis à l'eau,puis séchée sur $MgSO_4$ avant d'être concentrée à sec. Le résidu est recristallisé dans le pentane. On obtient 3,3g (0,0128 mole) de 3-(2'-chlorophényl) 4-bromo 1H-pyrazole, qui fond à 80°C (rendement 65%)(composé 94).

EXEMPLE 6 :

En opérant comme à l'exemple 5 mais en utilisant des réactifs appropriés, on a obtenu les dérivés de formule I, dans laquelle X est un atome de brome, rassemblés dans le tableau C suivant:

| COMPOSE N° | Y | Z | Rdt(%) | F(°C) ou analyse |
|---|---|---|---|---|
| 18 | F | H | 54 | RMN |
| 19 | H | Cl | 95 | 119 |
| 20 | Cl | Cl | 86 | 140 |
| 21 | $OCF_2$ | O | 50 | 144 |
| 95 | $NO_2$ | Cl | 44 | 190 |

EXEMPLE 7

On dissout, à température et sous agitation, 6,8g (0,038 mole) de 3-(2'-chlorophényl) 1H-pyrazole, préparé comme à l'exemple 9, dans 30ml d'acide acétique.On introduit ensuite 2,9g(0,0409 mole) de chlore dans le milieu réactionnel. L'agitation est maintenue pendant 1 heure à température ambiante puis l'acide acétique est chassé sous pression réduite. Le résidu est repris au chlorure de méthylène et cette solution organique est lavée à l'eau bicarbonatée, puis à l'eau, puis séchée sur $Na_2SO_4$ avant d'être concentrée à sec. Le résidu est chromatographié sur colonne de silice (éluant: heptane/acétate d'éthyle 60/40). On obtient 3,6g de 3-(2'-chlorophényl) 4-chloro 1H-pyrazole(composé 96), qui est un miel (rendement 44%), dont les caractéristiques sont annexées (H).

EXEMPLE 8 :

2,3 g (0,015 mole) de 3-(3-méthylphényl) 1H-pyrazole (préparé comme à l'exemple .. est dissout dans 45 ml de dichlorométhane, à température ambiante et sous agitation. On ajoute ensuite 2,1 g (0,016 mole) de N-chlorosuccinimide, puis on poursuit l'agitation 60 heures à température ambiante. Le mélange réactionnel est alors concentré puis chromatographié sur colonne de silice (éluant heptane/acétate d'éthyle 80/20). On obtient 1,4 g (0,007 mole) de 4-chloro-3-(3-méthylphényl) 1H-pyrazole(composé 97) qui fond à 109°C (rendement 50 %).

EXEMPLE 9: En opérant comme à l'exemple 8 en utilisant des réactifs appropriés, on a obtenu les dérivés de formule I, dans laquelle X est un atome de chlore, rassemblés dans le tableau D suivant :

| COMPOSE N° | Y | Z | Rdt(%) | F(°C) ou analyse |
|---|---|---|---|---|
| 24 | Cl | Cl | 53 | 140 |
| 25 | $OCF_2$ | O | 40 | 147 |
| 26 | $CH_3$ | H | 88 | RMN |
| 98 | $nC_3H_7$ | Cl | 47 | RMN |
| 99 | $CH_3$ | $CF_3$ | 68 | RMN |
| 100 | H | $OCF_3$ | 54 | 62 |
| 101 | $OCH_3$ | Cl | 44 | 70 |
| 102 | Cl | Br | 65 | 142 |
| 103 | H | $CF_3$ | 100 | 81 |

| | | | | |
|---|---|---|---|---|
| 104 | $C_2H_5$ | Cl | 83 | 84 |
| 105 | F | Br | 88 | 114 |
| 106 | H | CN | 79 | 110 |
| 107 | $O(CH_2)_2$ | O | 74 | RMN |
| 108 | $CF_3$ | H | 40 | 111 |
| 109 | $SOCH_3$ | Cl | 41 | 171 |
| 110 | Cl | F | 94 | 105 |
| 111 | $CH_3$ | Cl | 28 | RMN |
| 112 | F | Cl | 46 | 120 |
| 113 | $OCH_2$ | O | 85 | 100 |
| 114 | F | $CF_3$ | 67 | 97 |
| 115 | $OCH_3$ | $OCH_3$ | 20 | 90 |
| 116 | F | F | 68 | 95 |
| 117 | $CH_3$ | H | 88 | RMN |
| 118 | H | F | 57 | 120 |
| 119 | naphty l | naphtyl | 77 | 170 |
| 120 | $NO_2$ | Cl | 80 | 186 |
| 121 | $C_6H_5$ $CH_2O$ | H | 89 | 91 |
| 122 | $CH_3$ | $CH_3$ | 33 | 89 |
| 123 | H | Cl | 13 | 122 |
| 124 | Cl | $NO_2$ | 70 | 162 |
| 125 | H | $NO_2$ | 85 | 148 |
| 127 | $NO_2$ | H | 91 | miel |
| 128 | H | Br | 80 | 151 |
| 129 | $(CH_3)_3Si$ $CH_2$ | Cl | 87 | 92 |
| 130 | $NO_2$ | $CH_3S$ | 60 | miel |
| 131 | $NO_2$ | $N(CH_3)_2$ | 40 | miel |
| 132 | $NH_2$ | Cl | 71 | 121 |
| 133 | Br | H | 92 | miel |
| 134 | $NO_2$ | $CH_3$ | 46 | 169 |
| 135 | $C_2H_5O$ | Cl | | 96 |
| 136 | $NO_2$ | $SC_6H_5$ | 8 | 164 |

EXEMPLE 10: 4-iodo-3-(2,2-difluoro-1,3-benzodioxol-4-yl)-4-iodo-pyrazole(composé 137)

On ajoute 1,03g(0,0046 mol)de N-iodosuccinimide à une solution de 1g(0,0045 mol)

du pyrazole 4H correspondant obtenu à l'exemple 13, dans 50ml de dichloroéthane. Le mélange réactionnel est agité pendant 12 heures à température ambiante puis concentré à sec. Le résidu obtenu est purifié par passage sur une colonne de silice avec un mélange heptane/acétate d'éthyle 7/3 comme éluant, pour obtenir une poudre blanche, de point de fusion 142°C, avec un rendement de 79,5%.

Exemple 11 3-(2-chlorophényl) -4-cyano-pyrazole :

On dissout 10g (0,0055 mol) de 2-chlorobenzoylacétonitrile à température ambiante et sous agitation dans 30 ml de N,N dimethylformamide diméthylacétal selon le mode opératoire décrit à l'exemple n°1 . 7,5 g (0,032 mol) du composé ainsi obtenu ( 3-(2-chlorophényl)-2-cyano-1-diméthylamino-prop-1-ène-3-one , rendement 96 %) sont dissous dans 80 ml d'acide acétique puis additionnés de 2 ml (0,04 mol) d'hydrate d'hydrazine selon le mode opératoire décrit à l'exemple n°1. On obtient après trituration dans l'heptane 4,1 g de 3-(2-chlorophényl)-4-cyano-pyrazole(composé 138):
Rendement 63 % PF 160 °C

Exemple 12: 4-carbethoxy-3-(2-chlorophényl) pyrazole(composé 139)

2,04g (0,01 mol) de 3-(2-chlorophényl)-4-cyano-pyrazole obtenu selon l'exemple n°3, additionnés de 2,4 ml d'éthanol à 95 % et de 1,1 ml d'$H_2SO_4$ concentré sont portés à reflux pendant 6 heures; Le milieu réactionnel est coulé dans l'eau puis extrait au $CH_2Cl_2$. La phase organique est séchée sur $MgSO_4$ puis concentrée.Le résidu obtenu est purifié par passage sur une colonne de silice ($CH_2Cl_2$/ MeOH 98/2) pour conduire à l'obtention d'une huile jaune pâle:
Rendement 36 % N° analyse RMN 42280

Exemple 13 : 3-(2-chlorophényl)-4-thiocarbamyl-pyrazole(composé 140)

A une solution de 10 ml de DMF saturée d'acide chlorhydrique gazeux, sont ajoutés successivement 1 g (0,005 mol) de 3-(2-chlorophényl)-4-cyano-pyrazole obtenu selon l'exemple 11 et 0,75g (0,01 mol) de thioacétamide. Le milieu réactionnel est porté à 100°C pendant 2 heures puis refroidi à température ambiante et coulé dans l'eau. Le milieu réactionnel est ensuite lavé par une solution aqueuse de bicarbonate de sodium puis extrait au $CH_2Cl_2$. Après séchage de la phase organique sur $MgSO_4$ et évaporation, le résidu obtenu est purifié par passage sur une colonne de silice (Heptane/ Acétate d'éthyle 1/1) pour conduire à l'obtention d'une poudre jaune pâle:
Rendement 36 % PF 215 °C

Exemple 14 : 4-cyano-3-(2,2-difluoro-1,3-benzodioxol-4-yl) pyrazole

(composé 141)
Le 3-oxo-3-(2,2-difluoro-1,3-benzodioxol-4-yl) propanonitrile intermédiaire est obtenu selon la méthode décrite dans Synthesis 1983,308 par JC. KRAUSS, TL. CUPPS, DS. WISE et LB. TOWNSEND par condensation de l'anion de l'acide cyanoacétique sur le chlorure de l'acide (2,2-difluoro-1,3-benzodioxol-4-yl) carboxylique obtenu selon le mode opératoire décrit dans le brevet EP 333658. On dissout ensuite 5g (0,022 mol) de 3-oxo-3-(2,2-difluoro-1,3-benzodioxol-4-yl) propanonitrile dans 10 ml de N,N dimethylformamide diméthylacétal et chauffés à 70 °C. Les 5,7 g (0,022 mol) du composé ainsi obtenu (2-cyano-3-(2,2-difluoro-1,3-benzodioxol-4-yl)-1-diméthylamino-prop-1-ène-3-one) sont dissous dans 50 ml d'acide acétique puis additionnés de 1,5 ml (0,025 mol) d'hydrate d'hydrazine . On obtient après passage sur une colonne de silice (Heptane/ Acétate d'éthyle 6/4) 2,42g d'une poudre jaune pâle:Rendement 44 % PF 175 °C.

Exemple 15 : 3-(2,2-difluoro-1,3-benzodioxol-4-yl)-4-formyl-pyrazole

(composé 142)
1,1g (0,0044 mol) de 4-cyano-3-(2,2-difluoro-1,3-benzodioxol-4-yl) pyrazole précédemment obtenu à l'exemple n°DC**6** sont dissous dans 10 ml de toluène sous atmosphère d'azote à -65°C et additionnés de 5,74 ml (0,0057 mol) de diisobutylaluminohydrure d'aluminium en solution dans le toluène. Après 30 minutes d'agitation à -70°C, le milieu réactionnel est porté progressivement à température ambiante et agité ainsi pendant 3 heures. Le milieu réactionnel est ensuite hydrolysé par une solution aqueuse saturée de chlorure d'ammonium puis par une solution aqueuse d'acide chlohydrique 10% jusqu'à pH 4. Après extraction à l'acétate d'éthyle, le phase organique est séchée sur $MgSO_4$ et évaporée. L'huile ainsi obtenue est triturée dans un mélange

heptane / éther diisopropylique pour conduire à l'obtention d'une poudre jaune.Rendement 50 % PF 115 °C.

Exemple 16 : 3-(2,2-difluoro-1,3-benzodioxol-4-yl)-4-méthyl-pyrazole (composé 143)

12g de 1-(2,2-difluoro-1,3-benzodioxol-4-yl) propanone sont préparés par condensation du N,N diméthylpropionamide sur l'anion lithié du 2,2-difluoro-1,3-benzodioxole obtenu selon le mode opératoire décrit dans le dans le brevet EP 333658 puis dissous dans 14,6(0,11 mol) de N,N dimethylformamide diméthylacétal et chauffés à 70 °C selon le mode opératoire décrit à l'exemple n°**1**. 25g (0,05 mol) d'ènaminone ainsi obtenue sont dissous dans 130 ml d'acide acétique puis additionnés de 3,3 ml (0,069 mol) d'hydrate d'hydrazine . Le produit brut obtenu est purifié par trituration dans le pentane.Rendement 27% PF 93°C

Exemple 17: 3-(2,2-difluoro-1,3-benzodioxol-4-yl)-4-nitro-pyrazole (composé 144)

A une solution de 3g (0,013 mol) de pyrazole (Y=H) obtenu selon l'exemple n°**1** précédemment décrit, dans 60 ml de dichloroéthane, sont ajoutés successivement à 0°C, 6 ml d'acide sulfurique concentré puis 1,92g (0,019 mol) de $KNO_3$ par portion. Le milieu réactionnel est ainsi agité à 0°C pendant 1 heure puis porté à températue ambiante et agité pendant 40 minutes. Le milieu réactionnel est précipité par addition de glace puis filtré pour conduire à l'obtention d'une poudre beige.Rendement 50 % PF 135°C.

Exemple 18 : Tetrafluoroborate de-3-(2,2-difluoro-1,3-benzodioxol-4-yl)-4-diazonium pyrazole(composé 145)

Le 3-(2,2-difluoro-1,3-benzodioxol-4-yl)-4-nitro-pyrazole précédemment obtenu à l'exemple 17 est réduit en 4-amino-3-(2,2-difluoro-1,3-benzodioxol-4-yl) pyrazole selon la méthode décrite par Vogel (Practical Organic Chemistry, fourth Edition p. 660: Fe/HCl) . 0.8g (0,003 mol) de 4-amino-3-(2,2-difluoro-1,3-benzodioxol-4-yl) pyrazole en solution dans 10 ml de THF anhydre sont additionnés à 0°C à une solution de 10 ml de THF contenant 1 ml(0,0081 mol) d'éthérate de trifluorure de Bore. 0.6 ml(0,005 mol) de tertiobutylnitrite en solution dans 5 ml de THF sont ensuite ajoutés au mélange réactionnel. L'agitation est maintenue 1 heure à 0°C puis le milieu réactionnel est dilué au pentane et filtré sur verre fritté. Après séchage, on obtient une poudre beige:Rendement 37 % PF 210°C.

Exemple 19 : 4-acétyl-3-(2,2-difluoro-1,3-benzodioxol-4-yl) pyrazole (composé 146)

8g (0,04 mol) de 4-acétyl-(2,2-difluoro-1,3-benzodioxole) obtenus selon le brevet EP 333658 en solution dans 20 ml d'éther sont ajoutés à une suspension de 3,2g ( 0,08 mol) d'hydrure de sodium à 60% dans 30 ml d'éther contenant 7g (0,08 mol) d'acétate d'éthyle. La fin de l'addition est poursuivie par un chauffage du mélange réactionnel au reflux pendant 2 heures. Le milieu réactionnel est dilué avec 50 ml d'éther puis additionné de 2 ml d'éthanol absolu et de 20 ml d'eau afin de détruire le NaH excédentaire. Le pH du milieu réactionnel est ensuite amené à 6 par addition d'une solution aqueuse d'acide chlohydrique 1N. Après extraction à l'éther, séchage sur $MgSO_4$, évaporation et purification par passage sur une colonne de silice (Heptane/Acétate d'éthyle 9/1) 5 g d'une poudre jaune pâle (1-(2,2-difluoro-1,3-benzodioxol-4-yl)1,3- butanone Rendement 52% PF 85°C).

2.04g (0,0084 mol) de ce dernier composé sont ensuite dissous dans 1,23 ml( 0,0092 mol) de N,N dimethylformamide diméthylacétal selon le mode opératoire décrit à l'exemple n°**1**et chauffés à 70 °C selon le mode opératoire décrit à l'exemple n°**1** puis additionnés après isolement de l'ènaminone intermédiaire de 0,43 ml (0,0092 mol) d'hydrate d'hydrazine selon le mode opératoire décrit à l'exemple n°**1**. Le composé désiré est séparé du milieu réactionnel par chromatographie sur colonne de silice (Heptane/Acétate d'éthyle 6/4):Rendement 23 % PF 108°C.

Exemple 20: 4-méthylthio-3-(2-nitro-3-chlorophényl)-pyrazole (composé 147)

7g (0,035 mol) de (2-nitro-3-chlorophényl) acétophénone , en solution dans 50 ml d'acide acétique sont additionnées de 1,81 ml (0,0354 mol de brome) à température ambiante. Après 12 heures d'agitation,l'évaporation de l'acide acétique conduit à l'obtention d'un précipité jaune: (2-nitro-3-chlorophényl)-bromoacétophénone: Rendement 85 %

1,5 g (0,0061 mol) de (2-nitro-3-chlorophényl)-méthylthioacétophénone sont préparés par addition à 0°C de 1,3 g (0,018 mol) de méthanethiolate de sodium en solution dans 10 ml de méthanol sur 4,7 g (0,0168 mol) de (2-nitro-3-chlorophényl) bromoacétophénone obtenus précédemment.

1,5 g (0,0061 mol) de (2-nitro-3-chlorophényl) thiométhylacétophénone sont dissous dans 1,6ml(0,012 mol) de N,N dimethylformamide diméthylacétal selon le mode opératoire décrit à l'exemple n°**1**et chauffés à 70 °C puis additionnés après isolement de l'ènaminone intermédiaire de 2 ml (0,042 mol) d'hydrate d'hydrazine selon le mode opératoire décrit à l'exemple n°**1**. Après purification par passage sur colonne de silice (Heptane/ Acétate d'éthyle 75/25) , 600 mg du composé désiré sont obtenus: Rendement 36% PF 169°C.

Exemple 21 ; 3-(3-bromophényl)-4-méthylsulfonyl-pyrazole (composé 147)

27,8g (0,1 mol) de 3-bromoacétophénone en solution dans 300 ml d'acétonitrile sont additionnées de 10,2g (0,1mol ) de méthylsulfinate de sodium et portés à reflux pendant 8 heures. Après refroidissement et évaporation de l'acétonitrile, le milieu réactionnel est lavé à l'eau et extrait au $CH_2Cl_2$.Le résidu brut obtenu est purifié par trituration dans l'éther diisopropylique et conduit à l'obtention d'une poudre jaune: (3-bromophényl) methylsulfonylacétophénone: Rendement 87 % PF 104°C

1,1 g (0,004 mol) de (3-bromophényl) methylsulfonylacétophénone sont dissous dans 20ml (0,14 mol) de N,N dimethylformamide diméthylacétal et chauffés à 70 °C puis additionnés après isolement de l'ènaminone intermédiaire de 0,3 ml (0,006 mol) d'hydrate d'hydrazine selon le mode opératoire décrit à l'exemple n°**1**. Après purification par trituration dans l'éther diisopropylique, on obtient une poudre beige : Rendement: 44% PF 140° C.

Exemple 22: 3-(2,2-difluoro-1.3-benzodioxol-4-yl)-4-thiocyanato-pyrazole(composé 148)

A une solution toluènique (80 ml) contenant 9,03 g (0,04 mol) de **3**-oxo-3-(2,2-difluoro-1,3-benzodioxol-4-yl) propanonitrile préparé selon l'exemple n°**6** et 3,8g (0,02) d'acide paratoluènesulfonique, sont ajoutés 2,3 ml (0,048 mol) d'hydrate d'hydrazine. Le mélange réactionnel est chauffé à 80°C pendant 2 heures. Après refroidissement à température ambiante, le milieu réactionnel est dilué à l'acétate d'éthyle, lavé à l'eau et par une solution saturée de chlorure de sodium. Après séchage sur $MgSO_4$, l'évaporation de la phase organique conduit à l'obtention d'une poudre blanche purifiée par trituration dans l'éther: 5-amino-3-(2,2-difluoro-1,3-benzodioxol-4-yl) pyrazole. Rendement 62% PF 152°C.

A une solution de 1,63 g (0,0168 mol ) de thiocyanate de potassium dans 15 ml méthanol refroidie à -70°C est ajouté 0,45 ml (0,0088 mol ) de brome en solution méthanolique (5 ml) préalablement refroidie à -70°C. 1,92 g (0,008 mol) de 5-amino-3-(2,2-difluoro-1,3-benzodioxol-4-yl) pyrazole en solution dans 5 ml de méthanol refroidi à -70°C est ensuite ajouté tout en maintenant la température du milieu réactionnel à -70°C. L'agitation est maintenue pendant 1h30 à -70°C puis environ 30 minutes à température ambiante. Le traitement du mélange réactionnel est réalisé par ajoût d'acétate d'éthyle, lavage à l'eau et par une solution saturée de chlorure de sodium. Après séchage sur $MgSO_4$, l'évaporation de la phase organique conduit à l'obtention de : 5-amino-3-(2,2-difluoro-1,3-benzodioxol-4-yl)-4-thiocyanato-pyrazole. Rendement 80 % PF 264°C.

A une solution de 1,95 g (0,0065 mol ) de 5-amino-4-thiocyanato-3-(2,2-difluoro-1,3-benzodioxol-4-yl) pyrazole dans 25 ml de THF à 0°C sont ajoutés 0,94 ml ( 0,008 mol) de tertiobutylnitrite en solution dans 5 ml de THF. Le mélange réactionnel est agité pendant 1h30 à température ambiante puis dilué à l'acétate d'éthyle et lavé à l'eau. Après séchage sur $MgSO_4$ et évaporation du solvant, le résidu obtenu est chromatographié sur colonne de silice et conduit à l'obtention de 3-(2,2-difluoro-1,3-benzodioxol-4-yl)-1-(N-tétrahydrofuranyl)-4-thiocyanato-pyrazole. Rendement 20% huile N° Analyse 44694.

0,33g (0,00094 mol) de l'aminal précédemment obtenu est déprotégé par dissolution à température ambiante dans 10 ml de méthanol additionnés de 0,033 g (0,00018 mol) d'acide paratoluènesulfonique. Après 1 heure d'agitation à à température ambiante, le milieu réactionnel est dilué à l'acétate d'éthyle et lavé par une solution saturée de bicarbonate de sodium. Le séchage sur $MgSO_4$ et l'évaporation de la phase organique conduit à l'obtention d'une poudre jaune pale:3-(2,2-difluoro-1,3-benzodioxol-4-yl)-4-thiocyanato-pyrazole: Rendement 98% PF 163°C.

Exemple 23: 1-Méthyl, 3-(2′,3′-Dichloro Phényl), 4-Chloro Pyrazole(composé 149)

On chauffe, à 150°C pendant 1 heure, 1,5 g de 4-chloro, 3-(2,3-dichloro) phénylpyrazole et 0,40 g de méthylphosphonate de diméthyle. Après retour à température ambiante, le milieu est repris avec une solution aqueuse saturée en $NaHCO_3$, extrait avec 2 x 50 ml de $CH_2Cl_2$. Les phases organiques rassemblées sont séchées sur $Na_2SO_4$, concentrées sous pression réduite.Le solide brut est purifié par chromatographie liquide sur colonne de silice (éluant: heptane/ ACOF [80/20])

Exemple 24 :Sont préparés comme à l'exemple 23, les 4-chloro-3(5)-phényle(substitués)-I-R-pyrazoles de formules I ou Ibis suivants:

| COMPOSE N° | Y | Z | R | I(%) | I bis (%) | F(°C) ou analyse |
|---|---|---|---|---|---|---|
| 150 | $OCF_2$ | O | $CH_3$ | 100 | | 60 |
| 151 | $OCF_2$ | O | $CH_3$ | | 100 | huile |
| 152 | Cl | Cl | $CH_3$ | | 100 | 110 |
| 153 | Cl | Cl | $CH_3$ | 100 | | huile |
| 154 | Cl | H | $CH_3$ | | 100 | 66 |

Exemple 25: 1-benzyl, 3(5)-(2',2'-difluoro,1',3'-dioxolano) phényl, 4-chloropyrazole(composé 155). :

On ajoute 1,00 g de 4-chloro, 3-(2',2'-difluro, 1',3'-dioxolano)phénylpyrazole et 0,75 g de bromure de benzyle dans 20 ml de méthanol absolu, à une solution de méthylate de sodium préparée à partir de 0, 10 g de sodium en morceaux et 10 ml de méthanol absolu, à température ambiante. Le milieu réactionnel est agité pendant 12 heures à température ambiante, concentré à sec sous pression réduite, repris avec 80 ml d'un mélange eau/acétate d'éthyle (1/1). La phase organique est séchée sur $MgSO_4$, concentrée sous pression réduite. Le solide brut est purifié par chromatographie liquide sur colonne de silice (éluant :Heptane/ acétate d'éthyle [80/20]).

| COMPOSE N° | Y | Z | R | I(%) | I bis (%) | F(°C) ou analyse |
|---|---|---|---|---|---|---|
| 156 | $OCF_2$ | O | $CH_2C_6H_5$ | 70 | 30 | huile |
| 157 | $OCF_2$ | O | $SO_2N(CH_3)_2$ | 100 | | 50 |

Exemple 26 : 1 (2'-cyano éthyl), 4-chloro, 3-(2',2'-difluoro-1,3'dioxolano)phényl pyrazole(composé 158)

On dissout, dans 20 ml de dioxanne, 1,00 g de 4-Chloro,3-(2',2'-Difluoro, 1',3'Dioxolano) phényl pyrazole et 0,20 g d'acrylonitrile. On ajoute 0,02 ml d'une solution de Triton B à 40 % dans le méthanol. Le milieu réactionnel est agité pendant 12 heures à température ambiante, concentré à sec sous pression réduite. Le solide résiduel est trituré avec 10 ml d'heptane, récupéré par filtration et séché sous pression réduite. point de fusion: 83°C

Exemple 27 : 1-[(triméthylsilyl)méthyl], 4-chloro,3-(2'-nitro, 3'-chloro)phényl pyrazole.(composé 159)

On ajoute 0,85 g de carbonate de potassium anhydre à une solution de 1,30 g de 4-chloro,3-(2'-nitro, 3'-Chloro)phényl pyrazole et 0,70 g de chlorure (triméthylsilyl) méthyle dans 30 ml de N,N-dimethylformamide. Le milieu réactionnel est agité pendant 12 heures à température ambiante, dilué avec 100 ml d'un mélange éther-$H_2O$ (1/1). La phase éthérée est séchée sur $MgSO_4$ et concentrée sous pression réduite.L'huile résiduelle est purifée par chromatographie liquide sur colone de silice (éluant: heptane-acétate d'éthyle [50/50]).

Exemple 28:

Sont préparés comme à l'exemple 27 les composés suivants :

| COMPOSE N° | Y | Z | R | I(%) | I bis (%) | F(°C) ou analyse |
|---|---|---|---|---|---|---|
| 160 | $NO_2$ | Cl | $CH_2S$-($pClC_6H_4$) | 80 | 20 | 88 |
| 161 | $NO_2$ | Cl | $CH_2SCH_3$ | 80 | 20 | 65 |
| 162 | $NO_2$ | Cl | $CH_2OCH_3$ | 100 | | 124 |
| 163 | $NO_2$ | Cl | $CH_2OCH_3$ | | 100 | 128 |
| 164 | $NO_2$ | Cl | $CH_2O(CH_2)_2OCH_3$ | 100 | | 57 |
| 165 | $NO_2$ | Cl | $CH_2O(CH_2)_2Si(CH_3)_3$ | 53 | 47 | huile |
| 166 | $NO_2$ | Cl | $CH_2SCN$ | 84 | 16 | 99 |
| 167 | $NO_2$ | Cl | $CH_2SO_2CH_3$ | 50 | 50 | 62 |
| 168 | $NO_2$ | Cl | $CH_2$(o-$NO_2$ $C_6H_5$) | | 100 | 115 |
| 169 | $NO_2$ | Cl | $CH_2CO$(4-$CH_3OC_6H_4$) | 100 | | 146 |
| 170 | $NO_2$ | Cl | $CH_2OCH_2C_6H_5$ | 80 | 20 | 84 |
| 171 | H | Cl | $CH_2CH_2Cl$ | 80 | 20 | huile |

Exemple 29 : 1-(chlorothioformyl), 4-chloro,3-(2′,2′-difluoro,1′3′-dioxolano) phényl pyrazole(composé 172).

On chauffe, au reflux du thiophosgène (70°C) pendant 2 heures, 1,00 g de 4-chloro,3-(2′,2′-difluoro,1′3′-dioxolano) phényl pyrazole et 0, 15 ml de thiophosgène en solution dans 50 ml de toluène. Le milieu réactionnel est concentré à sec sous pression réduite. Le résidu est purifié par chromatographie liquide sur colonne de silice (Heptane-AcOEt [90/10]). Point de fusion: 85°C.

Exemple 30 : 1-(Benzoyl), 4-chloro, 3-(2′,2′-difluoro, 1′,3′-dioxolano) phényl pyrazole(composé 173)

0,55 g de chlorure de benzoyle dilués dans 10 ml de THF anhydre, sont additionnés, goutte à goutte, à une solution refroidie à + 10°C de 1,0 g de 4-Chloro, 3-(2′,2′-Difluoro,1′,3′-Dioxolano) Phényl Pyrazole; 0,08 g de DMAP et 0,55 ml de triéthylamine dans 20 ml de THF anhydre.
L'agitation est poursuivie 2 heures à température ambiante. Le milieu réactionnel est concentré à sec sous pression réduite, repris avec 80 ml d'un mélange $H_2O$-acétate d'éthyle (1/1). La phase organique est séchée sur $MgSO_4$ et concentrée sous pression réduite.Point de fusion: 85°C.

Exemple 31

Sont préparés comme à l'exemple 30 les composés suivants:

| COMPOSE N° | Y | Z | R | F(°C) ou analyse |
|---|---|---|---|---|
| 174 | $OCF_2$ | O | $COCH_3$ | 125 |
| 175 | $CH_3$ | Cl | $COCH_3$ | 63 |
| 176 | $NO_2$ | Cl | $COCH_3$ | |

Exemple 32: 1-(Methoxycarbonyl), 4-chloro,3-(2′,2′-difluoro,1′,3′-dioxolano) phényl pyrazole(composé 177)

5 ml de chloroformiate de méthyle dilués dans 10 ml de THF ahnhydre sont additionnés, goutte à goutte, à une solution refroidie à 0°C de 1,0 g de 4-Chloro,3-(2′,2′-Difluoro, 1′,3′-Dioxolano) Phényl Pyrazole, 0,08 g de DMAP et 0,55 mg de triéthylamine dans 20 ml de THF anhydre.
L'agitation est poursuivie 2 h à température ambiante. Le milieu réactionnel est concentré à sec sous pression réduiteet, repris avec 80 ml d'un mélange $H_2O$-AcOEt (1/1). La phase organique est séchée sur $MgSO_4$ et concentrée sous pression réduite. Point de fusion:75°C.
En opérant comme précédemment, on obtient le 1-(benzyloxycarbonyl), 4-chloro,3-(2′,2′-difluoro, 1′,3′-dioxolano) phényl pyrazole(composé 178) ; point de fusion: 83°C.

Exemple 33 : 1-(t-Butyloxycarbonyl), 4-chloro,3-(2′,2′-difluoro,1′,3′-dioxolano) phényl pyrazole.(composé 179)

1,00 g de di(t-Buyryloxycarbonyl) anhydre dilués dans 10 ml d'acétonitrile sont additionnées, goutte à goutte, à une solution de1,0 g de 4-chloro,3-(2′,2′-Difluoro, 1′,3′-Dioxolano) Phényl Pyrazole, 0,045 g de DMAP et 0,55 ml de triéthylamine dans 20 ml de'acétonitrile.L'agitation est poursuivie pendant 2 heures à température ambiante. Le milieu réactionnel est concentré à sec sous pression réduite. Le solide résiduel est purifié par chromatographie liquide sur colonne de silice (éluant: heptane-acétate d'éthyle [80/20]). Le solide a la consistance d'une meringue.

Exemple 34: 1-(Phénoxycarbonyl), 4-chloro,3-(2′-nitro, 3′-chloro) phényl pyrazole(composé 180)

On ajoute, par petites fractions, 1,0 ml de chloroformiate de phényle, à une solution refroidie à 0°C de 1,3 g de 4-chloro,3-(2′-nitro, 3-chloro) phénylpPyrazole dans 20 ml de pyridine. L'agitation est poursuivie 8 heures à température ambiante. Le milieu réactionnel est concentré à sec sous pression réduite et repris avec 100 ml d'un mélange $H_2O$ -acétate d'éthyle 1/1). La phase organique est séchée sur Mg $SO_4$ et concentrée sous pression réduite. Le solide résiduel est purifié par recristallisation dans l'éther de diisopropyle. Point de fusion: 123°C.

EXEMPLE 35 Sont préparés comme à l'exemple 34 les composés suivants:

| COMPOSE N° | Y | Z | R | F(°C) ou analyse |
|---|---|---|---|---|
| 181 | $NO_2$ | Cl | C(O)S $C_2H_5$ | 109 |

| | | | | |
|---|---|---|---|---|
| 182 | $NO_2$ | Cl | $CO_2C(CH_3)_2$ $CO_2C_2H_5$ | 219 |
| 183 | $NO_2$ | Cl | $CO_2CHCH_2$ | 118 |
| 184 | $NO_2$ | Cl | $CO_2$(p-$NO_2$ $C_6H_4$) | 185 |
| 185 | $NO_2$ | Cl | $CO_2CH_2CH$ $Cl_2$ | 130 |

Exemple 36 : 1-(Isopropylaminocarbonyl),4-chloro,3-(2',2'-difluoro,1',3'-dioxolano) phényl pyrazole.(composé 186)

On ajoute, goutte à goutte, 0,45 g d'isocyanate d'isopropyle à une solution de 1,0 g de 4-chloro,3-(2',2'difluoro, 1',3'-dioxolano) phényl Pyrazole et 0,50 g de triéthylamine dans 20 ml de DMF anhydre. L'agitation est poursuivie pendant 2 heures à température ambiante. Le traitement est identique à celui de l'exemple 32. Point de fusion: 135°C.

Exemple 37: 1-(4'-Méthyl phénylsulfonyl); 4-chloro, 3-(2',2'-difluoro,1',3'-dioxolano) phényl pyrazole.(composé 187)

On ajoute 0,75 g de chlorure de tosyle, par petites fractions, à une solution de 1,0 g de 4-chloro,3-(2',2'-difluoro,1',3'-dioxolano) phényl pyrazole et 0,5 ml de pyridine dans 20 ml de toluène. L'agitation est poursuivie pendant 2 heures à 40°C. Le traitement est identique à celui de l'exemple 32. Le solide résiduel est purifié par chromatographie liquide sur colonne de silice (éluant: heptane-acétate d'éthyle [80/20]).Point de fusion: 100°C.

EXEMPLE 38 : Test in vivo sur Botrytis cinerea sur feuille excisée de tomate (souches sensibles et résistantes aux benzimidazoles) :

On prépare, par broyage fin, une suspension aqueuse de la matière active à tester ayant la composition suivante:
- matière active: 60 mg
- agent tensioactif Tween 80, oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Des tomates cultivées en serre (variété Marmande) agées de 30 jours sont traitées par pulvérisation avec des suspensions aqueuses telles que définies ci-dessus et à diverses concentrations du composé à tester.

Au bout de 24 heures les feuilles sont coupées et mises dans une boîte de Pétri(diamètre 14 cm), dont le fond a été préalablement garni d'un disque de papier filtre humide (10 folioles par boîte).

L'inoculum est ensuite apporté à l'aide d'une seringue par dépôt de gouttes( 3 par foliole) d'une suspension de spores de Botrytis cinerea, sensibles aux benzimidazoles ou résistants aux benzimidazoles, obtenue à partir de cultures de 15 jours, mises ensuite en suspension à raison de 150 000 unités par $cm^3$.

Le contrôle est fait 6 jours après la contamination en comparaison avec un témoin non traité.

Dans ces conditions, on observe, à la dose de 1 g/l, une protection bonne ( au moins 75%) ou totale avec les composés suivants :
- Botrytis sensibles aux benzimidazoles: 9, 13, 17, 19, 21, 22, 23, 24, 25, 71, 73, 74, 88, 95, 99, 100, 104, 105, 107, 108, 113, 114, 116, 120, 122, 123, 124, 128, 133, 135, 147, 166, 172, 173, 175, 176, 177, 179, 180, 181, 182, 183, 184, 185.

EXEMPLE 39 : Test in vivo sur Piricularia oryzae responsable de la piriculariose du riz :

On prépare, par broyage fin, une suspension aqueuse de la matière active à tester ayant la composition suivante :

- matière active : 60 mg
- agent tensioactif Tween 80, oléate de dérivé polyoxyéthyléné du sorbitan) dilué à 10% dans l'eau: 0,3 ml
- on complète à 60 ml d'eau.

Cette suspension aqueuse est ensuite diluée par de l'eau pour obtenir la concentration désirée en matière active.

Du riz, semé en godets dans un mélange 50/50 de tourbe enrichie et de pouzzolane, est traité au stade 10 cm de hauteur par pulvérisation de la suspension aqueuse ci dessus.

Au bout de 24 heures, on applique sur les feuilles une suspension aqueuse de spores de Piricularia oryzae, obtenue à partir d'une culture de 15 jours, mise ensuite en suspension à raison de 100 000 unités par $cm^3$.

Les plants de riz sont placés pendant 24 heures en incubation (25°C, 100% d'humidité relative) , puis mis en cellule d'observation, dans les mêmes conditions, pendant 7 jours.

La lecture se fait 6 jours après la contamination.

Dans ces conditions, on observe, à la dose de 1 g/l, une protection bonne (au moins 75%) ou totale avec les composés suivants:

12, 17, 19, 21, 24, 25, 72, 78, 81, 84, 87, 95, 99, 100, 101, 103, 104, 105,.107, 108, 110, 111, 112, 113, 114, 116, 117, 118, 120, 122, 123, 124, 127, 128, 129, 130, 133, 134, 135, 141, 166, 172, 173, 174, 175, 176, 177, 179, 180, 181, 182, 183, 184, 185.

EXEMPLE 40 : Test in vivo sur Plasmopara viticola :

On prépare, par broyage fin, une suspension aqueuse de la matière active à tester ayant la composition suivante:

- matière active: 60 mg
- agent tensioactif Tween 80, oléate de dérivé polyoxyéthyléné du sorbitan) dilué à 10% dans l'eau : 0,3 ml
- on complète à 60 ml d'eau.

Cette suspension aqueuse est ensuite diluée par de l'eau pour obtenir la concentration désirée en matière active.

Des boutures de vigne (Vitis vinifera), variété Chardonnay, sont cultivées dans des godets. Lorsque ces plants sont âgés de 2 mois (stade 8 à 10 feuilles, hauteur de 10 à 15 cm), ils sont traités par pulvérisation au moyen de la suspension aqueuse ci-dessus.

Des plants, utilisés comme témoins sont traités par une solution aqueuse ne contenant pas la matière active.

Après séchage pendant 24 heures, on contamine chaque plant par pulvérisation d'une suspension aqueuse de spores de Plasmopara viticola obtenue à partir d'une culture de 17 jours, mise ensuite en suspension à raison de 100 000 unités par $cm^3$.

Les plants contaminés sont ensuite mis en incubation pendant deux jours à 18°C environ, en atmosphère saturée d'humidité puis pendant 5 jours à 20-22°C environ sous 90-100% d'humidité relative.

La lecture se fait 7 jours après la contamination, en comparaison avec les plants témoins.

Dans ces conditions, on observe, à la dose de 1 g/l, une protection bonne (au moins 75%) ou totale avec les composés suivants: 13, 19, 20, 21, 22, 24, 25, 64, 74, 77, 80, 81, 82, 102, 104, 106, 107, 109, 111, 113,125, 127, 131, 133, 134, 139, 142, 144, 186.

Ces résultats montrent clairement les bonnes propriétés fongicides des dérivés selon l'invention contre les maladies fongiques des plantes dues à des champignons appartenant caux familles les plus diverses telles que les Phycomycètes, les Basidiomycètes, les Ascomycètes , les Adelomycètes ou Fungi imperfecti, en particulier les Botrytis sp., Piricularia oryzae, Alternariale mildiou de la vigne.

En effet pour leur emploi pratique, les composés selon l'invention sont rarement utilisés seuls. Le plus souvent ces composés font partie de compositions. Ces compositions, utilisables comme agents herbicides, contiennent comme matière active un composé selon l'invention tel que décrit précédemment en mélange avec les supports solides ou liquides, acceptables en agriculture et les agents tensio-actifs également acceptables en agriculture. En particulier sont utilisables les supports inertes et usuels et les agents tensio-actifs usuels. Ces compositions font également partie de l'invention.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... Plus généralement les composés utilisés dans l'invention peuvent être combinés à tous les additifs solides ou liquides correspondant aux techniques habituelles de la mise en formulation.

D'une façon générale, les compositions selon l'invention contiennent habituellement de 0,05 à 95 % environ (en poids) d'un composé selon l'invention, un ou plusieurs supports solides ou liquides et, éventuellement, un ou plusieurs agents tensioactifs.

Par le terme "support", dans le présent exposé, on désigne une matière organique ou minérale, naturelle ou synthétique, avec laquelle le composé est combiné pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau ; alcools, notamment le butanol etc...).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque le composé et/ou le support inerte ne sont pas solubles dans l'eau et que l'agent vecteur de l'application est l'eau.

Ainsi donc, les compositions à usage agricole selon l'invention peuvent contenir les matières actives selon l'invention dans de trés larges limites, allant de 0,05 % à 95 % (en poids). Leur teneur en agent tensio-actif est avantageusement comprise entre 5 % et 40 % en poids.

Ces compositions selon l'invention sont elles-mêmes sous des formes assez diverses, solides ou liquides.

Comme formes de compositions solides, on peut citer les poudres pour poudrage (à teneur en composé pouvant aller jusqu'à 100 %) et les granulés, notamment ceux obtenus par extrusion, par compactage, par imprégnation d'un support granulé, par granulation à partir d'une poudre (la teneur en composé dans ces granulés étant entre 0,5 et 80 % pour ces derniers cas).

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active.

En plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précédemment cités.

A partir de ces concentrés, on peut obtenir par dilution avec de l'eau des émulsions de toute concentration désirée, qui conviennent particulièrement à l'application sur les cultures.

A titre d'exemple, voici la composition de quelques concentrés émulsionnables :

Exemple CE 1 :

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique q.s.p. | 1 litre |

Selon une autre formule de concentré émulsionnable, on utilise :

Exemple CE 2

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici une composition de suspension concentrée :

Exemple SC 1 :

| | |
|---|---|
| - composé | 500 g |
| - phosphate de tristyrylphénol polyéthoxylé | 50 g |
| - alkylphénol polyéthoxylé | 50 g |
| - polycarboxylate de sodium | 20 g |
| - éthylène glycol | 50 g |
| - huile organopolysiloxanique (antimousse) | 1 g |
| - polysaccharide | 1,5 g |
| - eau | 316,5 g |

Les poudres mouillables (ou poudre'à pulvériser) sont habituellement préparées de manière qu'elles contiennent 20 à 95 % de matière active, et elles contiennent habituellement, en plus du support solide, de 0 à 30 % d'un agent mouillant, de 3 à 20 % d'un agent dispersant, et, quand c'est nécessaire, de 0,1 à 10 % d'un ou plusieurs stabilisants et/ou autres additifs, comme des agents de pénétration, des adhésifs, ou des agents antimottants, colorants, etc...

Pour obtenir les poudres à pulvériser ou poudres mouillables, on mélange intimement les matières actives dans les mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables trés avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

A titre d'exemple, voici diverses compositions de poudres mouillables (ou poudres à pulvériser) :

Exemple PM 1

| | |
|---|---|
| - matière active (composé n°1) | 50 % |
| - alcool gras éthoxylé (agent mouillant) | 2,5 % |
| - phényléthylphénol éthoxylé (agent dispersant | 5 % |
| - craie (support inerte) | 42,5 % |

Exemple PM 2 :

| | |
|---|---|
| - matière active (composé n° 1) | 10 % |
| - alcool synthétique oxo de type ramifié, en C13 éthoxylé par 8 à 10 oxyde d'éthylène (agent mouillant) | 0,75 % |
| - lignosulfonate de calcium neutre (agent dispersant) | 12 % |
| - carbonate de calcium (charge inerte) .q.s.p. | 100 % |

Exemple PM 3 :

Cette poudre mouillable contient les mêmes ingrédients que dans l'exemple précédent, dans les proportions ci-après :

| | |
|---|---|
| - matière active | 75 % |
| - agent mouillant | 1,50 % |
| - agent dispersant | 8 % |
| - carbonate de calcium (charge inerte) q.s.p. | 100 % |

Exemple PM 4 :

| | |
|---|---|
| - matière active (composé n°1) | 90 % |
| - alcool gras éthoxylé (agent mouillant) | 4 % |
| - phényléthylphénol éthoxylé (agent dispersant) | 6 % |

Exemple PM 5 :

| | |
|---|---|
| - matière active (composé n°1) | 50 % |
| - mélange de tensio-actifs anioniques et non ioniques (agent mouillant) | 2,5 % |
| - lignosulfonate de sodium (agent dispersant) | 5 % |
| - argile kaolinique (support inerte) | 42,5 % |

Les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les composés selon l'invention peuvent être formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000 et de préférence entre 300 et 1500 microns.

La teneur en matière active de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale ou, de préférence, organique. On a obtenu d'excellents résultats avec l'urée. Dans le cas d'une charge insoluble, celle-ci est de préférence minérale, comme par exemple le kaolin ou la bentonite. Elle est alors avantageusement accompagnée d'agents tensio-actifs (à raison de 2 à 20 % en

poids du granulé) dont plus de la moitié est, par exemple, constituée par au moins un agent dispersant, essentiellement anionique, tel qu'un polynaphtalène sulfonate alcalin ou alcalino terreux ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

Exemple F 6 : Granulés dispersibles

Dans un mélangeur, on mélange 90 % en poids de matière active (composé n°1) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple F 7 : Granulés dispersibles

Dans un mélangeur, on mélange les constituants suivants:

- matière active (composé n° 1) ........... 75 %
- agent mouillant (alkylnaphtalène sulfonate de sodium ............................... 2 %
- agent dispersant (polynaphtalène sulfonate de sodium) .............................. 8 %
- charge inerte insoluble dans l'eau (kaolin) 15 %

Ce mélange est granulé en lit fluide, en présence d'eau, puis séché, concassé et tamisé de manière à obtenir des granulés de dimension comprise entre 0,15 et 0,80 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

En ce qui concerne les compositions adaptées au stockage et au transport, elles contiennent plus avantageusement de 0,5 à 95 % (en poids) de substance active.

L'invention a également pour objet l'utilisation des composés selon l'invention pour la lutte contre les maladies fongiques des plantes par traitement préventif ou curatif de ces dernières ou de leur lieu de croissance.

## Revendications

**1)** Dérivés 3-phénylpyrazoles, caractérisés en ce qu'ils sont de formules I et/ou I bis :

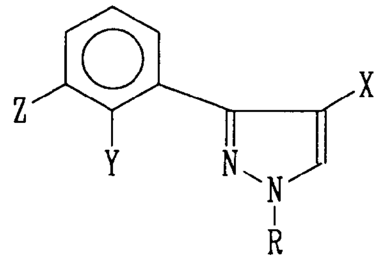

I

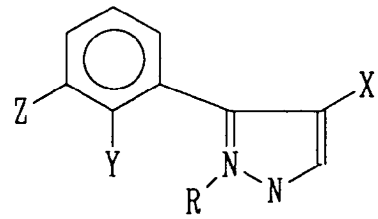

I bis

dans lesquelles:

**X** est:

- un atome d'hydrogène ou d'halogène,
- un groupe nitro, cyano, thiocyanato,
- un groupe alkyle, alkényle, alkynyle, alkoxy ou alkylthio, chacun de ces groupes étant ou non halogéné,
- un phényle ou phénoxy éventuellement substitué,
- un amino substitué ou non par un ou deux alkyles ou phényles,
- un groupe alkylcarbonyle, carbamoyle, carboxyle ou benzoyle
- un groupe alkylsulfonyle ou alkylsulfonyle, étant entendu que la partie alkyle de tous les groupes ci-dessus comprend de 1 à 4 atomes de carbone;

**Y et Z** , identiques ou différents, sont:

- un atome d'hydrogène, mais pas ensemble, ou d'halogène, un groupe hydroxy, nitro, nitroso, cyano, thiocyanato, amino substitué ou non par un ou deux alkyles ou phényles,
- un groupe alkyle, alkoxy ou alkylthio, hydroxyalkyle, alkényle, alkynyle, alkylsulfinyle, alkylsulfonyle, la partie alkyle de ces groupes comprenant de 1 à 4 atomes de carbone et pouvant être halogénée;
- phényle, phényloxy ou phénylthio ou benzyle substitué ou non sur le noyau,
- un groupe formyle, acétyle, alkyl- ou alkoxy (thio)carbonyle, mono ou di alkylamino-carbonyle ou -thio, carboxyle, carboxylate, carbamoyle, la partie alkyle de ces groupes comprenant de 1 à 4 atomes de carbone et pouvant être substituée par au moins un atome d'halogène;
- ou benzoyle, substitué ou non sur le noyau;

Y et Z peuvent également être reliés entre eux par un pont carboné comprenant de 1 à 4 atomes, dont au moins un peut être remplacé par un un atome d'oxygène, de soufre ou d'azote, les carbones de ce pont pouvant en outre être ou non substitués par au moins un atome d'halogène et/ou au moins un groupe alcoyle, alcoxy ou alcoylthio tels que définis ci-dessus, et pouvant chacun encore être reliés, par une double liaison, à un atome d'oxygène;

**R** est:

**-a)** un atome d'hydrogène ou d'halogène, un groupe nitro, amino, hydroxy, cyano, alkyle ou haloalkyle comprenant chacun de 1 à 6 atomes de carbone, un phényle substitué ou non par au moins un atome d'halogène, un groupe nitro ou haloalkyle de 1 à 3 atomes de carbone;

**- b) un groupe $S(O)_m$-$R_1$**, dans lequel:

- m est un entier de zéro à deux, et
- **$R_1$** est:
  - soit, quand m est égal à zéro:
    - un groupe haloalkyle de 1 à 6 atomes de carbone,
    - un phényle ou 3-pyridyl, chacun pouvant être substitué par au moins un atome d'halogène, un groupe nitro, alkyle, halo alkyle, alkoxy, haloalkoxy, la partie alkyle de ces quatre groupes comprenant de 1 à 4 atomes de carbone;
  - soit, quand m est égal à deux:
    - un groupe alkyle ou alkoxy, chacun comprenant de 1 à 6 atomes de carbone et étant ou non substitué par un ou plusieurs atomes d'halogène ou groupe alkoxy de 1 à 3 atomes de carbone; ou cycloalkyle de 3 à 6 atomes de carbone;
    - un groupe alkényle ou alkynyle ou alkénoxy, chacun comprenant de 3 à 6 atomes de carbone;
    - un groupe phényle, substitué ou non par 1 à 5 substituants choisis dans le groupe comprenant un atome d'halogène, un groupe nitro, un alkyle, haloalkyle, alkoxy, halohalkoxy comprenant de 1 à 4 atomes de carbone;

**c) un groupe $CH_2$-$NR_2R_3$**, dans lequel:

$R_2$ est un groupe:

- alkyle de 1 à 6 atomes de carbone, éventuellement substitué par un substituant choisi dans le groupe comprenant cyano, alkoxy, cycloalkyle de 3 à 7 atomes de carbone, alkylcarbonyle, alkoxycarbonyle mono- ou dialkylaminocarbonyle, alkylsulfinyle, alkylsulfonyle, dialkylaminoalkyle, la partie alkyle de ces groupes comprenant de 1 à 6 atomes de carbone;
- alkényle ou alkynyle de 2 à 6 atomes de carbone;
- cycloalkyle de 3 à 7 atomes de carbone;
- phényle ou benzyle éventuellement substitué par un substituant choisi dans le groupe comprenant un atome d'halogène, un groupe cyano, alkyle, alkoxy, haloalkyle ou haloalkoxy de 1 à 9 atomes d'halogène, alkylcarbonyle, alkoxycarbonyle, la partie alkyle de ces groupes comprenant de 1 à 6 atomes de carbone;

$R_3$ est un groupe:

- Het, Het-alkyle(de 1 à 6 atomes de carbone), Het-alkényle ou Het-alkynyle(chacun de 3 à 6 atomes de carbone), éventuellement substitué par un substituant choisi dans le groupe comprenant un atome d'halogène, un groupe cyano, alkyle, alkoxy, haloalkyle ou haloalkoxy de 1 à 9 atomes d'halogène, alkylcarbonyle, alkoxycarbonyle, la partie alkyle de ces groupes comprenant de 1 à 6 atomes de carbone;
  dans lequel Het est un radical hétérocyclique de 5 à 7 atomes, dont 1 à 3 hétéroatomes (azote,oxygène, soufre), éventuellement substitué par un substituant choisi dans le groupe comprenant un atome d'halogène, un groupe cyano, alkyle, alkoxy, haloalkyle ou haloalkoxy de 1 à 9 atomes d'halogène, alkylcarbonyle, alkoxycarbonyle, la partie alkyle de ces groupes comprenant de 1 à 6 atomes de carbone;
- dialkylaminoalkyle, la partie alkyle de ces groupes comprenant de 1 à 6 atomes de carbone; cycloalkyle ou cycloalkylalkyle(alkyle de 1 à4 atomes de carbone) de 3 à 7 atomes de carbone; ou phénéthyle éventuellement substitué par un substituant choisi dans le groupe comprenant un atome d'halogène, un groupe cyano, alkyle ou alkoxy chacun de 1 à 4 atomes de carbone;
  **$R_2$ et $R_3$ peuvent en outre former**, avec l'atome d'azote auquel ils sont reliés, un cycle azoté à 6 atomes, dont 4 sont des atomes de carbone éventuellement substitué et le cinquième est un atome de carbone ou un hétéroatome tel que oxygène, soufre, azote pouvant être substitué par un groupe alkyle, de 1 à 6 atomes de carbone, le cycle azoté pouvant lui même être substitué par un ou deux substituants choisis dans le groupe comprenant cyano, alkylcarbonyle, alkoxycarbonyle, mono- ou dialkylaminocarbonyle, alkylsulfinyle, alkylsulfonyle, la partie alkyle de ces groupes comprenant de 1 à 6 atomes de carbone, phénylsulfinyle, phénylsulfonyle;

**d) un groupe $(CH_2)_m$- $R_4$,** dans lequel:

m est égal à 1 ou 2,

$R_4$ est un groupe cyano, nitro, alkylcarbonyle, phénylcarbonyl, alkoxycarbonyl, mono- ou dialkylaminocarbonyle, $P(O)(alkoxy)_2$, $P(O)(benzyloxy)_2$, $P(O)(phénoxy)_2$, trialkylsilyle, phényle, étant entendu que le radical alkyle de ces groupes comprend de 1 à 4 atomes de carbone et est éventuellement halogéne et que le noyau phényle des radicaux aromatiques peut être substitué par 1 à 5 substituants choisis dans le groupe comprenant un atome d'halogène, un groupe nitro ou un radical alkyle, alkoxy, haloalkyle, haloalkoxy, alkoxycarbonyle, la partie alkyle de chacun de ces radicaux comprenant de 1 à 4 atomes de carbone;

**e) un groupe CH(R5)-X-R6,** dans lequel:

- $R_6$ est un atome d'hydrogène ou un alkyle de1 à 4 atomes de carbone,
- X est un atome d'oxygène ou un groupe $S(O)_n$, dans lequel:
- n est un entier égal à zéro ou deux,
- $R_5$ est:
  - alkyle de 1 à 4 atomes de carbone éventuellement substitué par un substituant choisi dans le groupe comprenant un atome d'halogène, un groupe cyano, alkoxy , phénoxy, benzyloxy, trialkylsilyl( la partie alkyle de chacun de ces radicaux comprenant de 1 à 4 atomes de carbone et les noyaux phényle peuvent être substitués par 1 à 5 substituants choisis dans le groupe comprenant un atome d'halogène, un groupe nitro ou un radical alkyle, alkoxy, haloalkyle, haloalkoxy,( la partie alkyle de chacun de ces radicaux comprenant de 1 à 4 atomes de carbone);
  - alkényle ou alkynyle de 3 à 6 atomes de carbone;
  - phényle ou benzyle pouvent être substitués par 1 à 5 substituants choisis dans le groupe comprenant un atome d'halogène, un groupe nitro ou un radical alkyle, alkoxy, haloalkyle, haloalkoxy,(1 à 4 atomes de carbone);

**f) un groupe $CHR_7R_8$,** dans lequel:

- $R_7$ est un atome d'hydrogène, un groupe haloalkyle ou alkoxy, chacun de 1 à 4 atomes de carbone,
- $R_8$ est un atome d'halogène, un groupe hydroxy, alkoxy ou O-C(O)-$R_9$ avec
- $R_9$ étant un atome d'hydrogène, un groupe alkyle, haloalkyle, alkényle 1 à 4 atomes de carbone, tetrahydrofuryle, tetrahydropyranyle ou alkoxycarbonyle,

  la partie alkyle de chacun de ces radicaux comprenant de1 à 6 atomes de carbone;

**g) un groupe C(X)-$R_{10}$**, dans lequel:
- X est un atome d'oxygène ou de soufre,
- $R_{10}$ est:
  - un atome d'hydrogène ou d'halogène,
  - un groupe alkyle de1 à 6 atomes de carbone, éventuellement substitué par un substituant choisi dans le groupe comprenant un atome d'halogène, un groupe cyano, nitro, alkylcarbonyle, alkoxycarbonyle, mono- ou dialkylaminocarbonyle, la partie akyle de chacun de ces radicaux comprenant de 1 à 4 atomes de carbone; un groupe cycloalkyle de3 à 6 atomes de carbone;
  - un groupe alkényle ou alkynyle, de 3 à 6 atomes de carbone, éventuellement substitué par un phényle pouvant être substitué par 1 à 5 substituants choisis dans le groupe comprenant un atome d'halogène, un groupe nitro ou un radical alkyle, alkoxy, haloalkyle, haloalkoxy, la partie alkyle de chacun de ces radicaux comprenant de1 à 4 atomes de carbone;
  - un groupe phényle, benzyle, 2-pyridyle, 3-pyridyle, 4-pyridyle, les noyaux pouvant être substitués par 1 à 5 substituants choisis dans le groupe comprenant un atome d'halogène, un groupe nitro ou cyano, ou un radical alkyle, alkoxy, haloalkyle, haloalkoxy, alkylcarbonyle ou alkoxycarbonyle, la partie alkyle de chacun de ces radicaux comprenant de1 à 4 atomes de carbone;
- un groupe CH($R_{11}$)-X-$R_{12}$, dans lequel:
  - $R_{11}$ est un atome d'hydrogène ou un alkyle de 1 à 4 atomes de carbone,
  - X est un atome d'oxygène ou le groupe $S(O)_p$, avec p égal à zéro ou 2;
  - $R_{12}$ est un alkyle de 1 à 4 atomes de carbone, éventuellement substitué par un atome d'halogène ou un alkoxy de 1 à 4 atomes de carbone; un groupe alkényle ou alkynyle, de 3 à 6 atomes de carbone; un groupe phényle ou benzyle pouvant être substitués par 1 à 5 substituants choisis dans le groupe comprenant un atome d'halogène, un groupe nitro ou un radical alkyle, alkoxy, haloalkyle, haloalkoxy,(1 à 4 atomes de carbone);
- un groupe CH($R_{11}$)-N$R_{13}R_{14}$, dans lequel $R_{13}$ et$R_{14}$, identiques ou différents, sont chacun:
  - un alkyle de 1 à 4 atomes de carbone, éventuellement substitué par un groupe cyano, alkylcarbonyle, alkoxycarbonyle ou dialkylaminocarbonyle,atome d'halogène ou un alkoxy de 1 à 4 atomes de carbone
  - un groupe phényle ou benzyle pouvent être substitué par 1 à 5 substituants choisis dans le groupe comprenant un atome d'halogène, un groupe nitro, cyano ou un radical alkyle, alkoxy, haloalkyle, haloalkoxy, alkoxycarbonyle, la partie alkyle de chacun de ces radicaux comprenant de1 à 4 atomes de carbone;
- un groupe CH$R_{11}$-$R_{15}$, dans lequel:
  - $R_{11}$ est comme défini ci-dessus et
  - $R_{15}$ est un groupe hétérocyclique $NC_4R_{16}R_{17}T$, dans lequel $R_{16}$ et-$R_{17}$, identiques ou différents, sont un atome d'hydrogène, un groupe alkyle ou alkoxycarbonyle chacun de 1 à 3 atomes de carbone, et T est un atome d'oxygène ou de soufre, un groupe carbonyle ou N-$R_{18}$, dans lequel $R_{18}$ est un atome d'hydrogène, un groupe alkyle, formyle,alkylcarbonyle ou alkoxycarbonyle, la partie alkyle de chacun de ces radicaux comprenant de1 à 4 atomes de carbone;

**h) un groupe-C(O)-X-$R_{19}$**, dans lequel:
- X est un atome d'oxygène ou de soufre,
- $R_{19}$ est:
  - un groupe alkyle de1 à 6 atomes de carbone, éventuellement substitué par un substituant choisi dans le groupe comprenant un atome d'halogène, un groupe cyano; un groupe cycloalkyle de 3 à 6 atomes de carbone, éventuellement substitué par un alkyle de 1 à 3 atomes de carbone; trialkylsilyle, phénylsulfonyle éventuellement substitué par au moins un atome d'halogène ou un groupe alkyle; alkoxycarbonyle, dialkylaminocarbonyle; la partie alkyle de chacun des radicaux précédents comprenant de 1 à 4 atomes de carbone; un groupe cycloalkyle de3 à 6 atomes de carbone;
  - un groupe cycloalkyle de3 à 6 atomes de carbone, éventuellement substitué par un alkyle de1 à 3 atomes de carbone,
  - un groupe alkényle ou alkynyle, de 2 à 6 atomes de carbone, éventuellement substitué par un phényle pouvant être substitué par 1 à 5 substituants choisis dans le groupe comprenant un atome

d'halogène, un groupe nitro ou un radical alkyle;

- un groupe phényle, benzyle, 2-pyridyle, 3-pyridyle, 4-pyridyle, les noyaux pouvant être substitués par 1 à 5 substituants choisis dans le groupe comprenant un atome d'halogène, un groupe nitro ou cyano, ou un radical alkyle, alkoxy, haloalkyle, haloalkoxy, alkylcarbonyle ou alkoxycarbonyle, alkylthio, alkylsulfinyle, alkylsulfonyle, la partie alkyle de chacun de ces radicaux comprenant de1 à 4 atomes de carbone;
- un groupe phénylalkyle ou un groupe hétérocyclylalkyle, dans lequel la partie alkyle comprend de1 à 4 atomes de carbone, et la partie héterocyclyle peut être 2-pyridyle, 3-pyridyle, 4-pyridyle, 2-furyl, 3-furyl, 2-thiényl, 3-thiényl, les noyaux pouvant être substitués par 1 à 5 substituants choisis dans le groupe comprenant un atome d'halogène, un groupe nitro, ou un radical alkyle, alkoxy, haloalkyle, haloalkoxy, alkylcarbonyle ou alkoxycarbonyle, alkylthio, alkylsulfinyle, alkylsulfonyle.

**i) un groupe $C(X)-NR_{20}R_{21}$**, dans lequel:

- X est un atome d'oxygène ou de soufre,
- $R_{20}$ et $R_{21}$, sont chacun:
  - un atome d'hydrogène ou un groupe alkyle de1 à 4 atomes de carbone, éventuellement substitué par un substituant choisi dans le groupe comprenant un atome d'halogène, un groupe cyano, alkylcarbonyle, alkoxycarbonyle, dialkylaminocarbonyle, la partie alkyle de chacun des radicaux précédents comprenant de 1 à 4 atomes de carbone;
  - un groupe cycloalkyle de 3 à 6 atomes de carbone, éventuellement substitué par un alkyle de1 à 3 atomes de carbone,
  - un groupe alkényle ou alkynyle, de 3 à 6 atomes de carbone,
  - un groupe phényle, benzyle, dont les noyaux peuvent être substitués par 1 à 5 substituants choisis dans le groupe comprenant un atome d'halogène, un groupe nitro ou cyano, ou un radical alkyle, alkoxy, haloalkyle, haloalkoxy, alkylcarbonyle ou alkoxycarbonyle, alkylthio, alkylsulfinyle, alkylsulfonyle, la partie alkyle de chacun de ces radicaux comprenant de1 à 4 atomes de carbone;

$R_{20}$ et $R_{21}$ peuvent en outre former, avec l'atome d'azote auquel ils sont reliés un cycleà 6 atomes, dont 4 sont des atomes de carbone éventuellement substitué et le cinquième est un atome de carbone ou un hétéroatome tel que oxygène, soufre,;

**j) un groupe $SiR_{22}R_{23}R_{24}$**, dans lequel $R_{22,}$ $R_{23}$ et $R_{24,}$ identiques ou différents, sont chacun un groupe alkyle, de1 à 4 atomes de carbone, ou un groupe phényle ou benzyle,

**k) un groupe $P(X)R_{25}R_{26}$**, dans lequel:

- X est un atome d'oxygène ou de soufre,
- $R_{25}$ et $R_{26,}$ identiques ou différents, sont chacun un groupe alkyle ou alkoxy, de1 à 4 atomes de carbone, ou un groupe phényle, phénoxy, benzyle ou benzoxy.

**2)** Dérivés selon la revendication 1, caractérisés en ce que, dans la formule, X est un atome de chlore ou de brome.

**3)** Dérivés selon l'une des revendications 1 et 2, caractérisés en ce que, dans la formule I, Y et/ou Z sont un atome d'hydrogène ou de chlore.

**4)** Dérivés selon l'une des revendications 1 et 2 , caractérisés en ce que Y et Z forment un pont comprenant 3 ou 4 atomes.

**5)** Dérivés selon l'une des revendications 1, 2 et 4, caractérisés en ce que Y et Z forment un pont méthylènedioxy éventuellement halogéné.

**6)** Dérivés selon l'une des revendications 1 à 5, caractérisés en ce que R est un atome d'hydrogène ou un groupe alkyl(1 à 3 atomes de carbone)carbonyle, alkyl(1 à 3 atomes de carbone)oxycarbonyle, phénylcarbonyle ou phényloxycarbonyle.

**7)** Procédé de préparation de dérivés selon l'une des revendications 1 à 6, caractérisé en ce qu'on fait réagir un phénylpyrazole de formule II, dans laquelle Y et Z ont les mêmes significations , avec un agent d'halogénation.

**8)** Procédé selon la revendication 7, caractérisé en ce qu'on fait agir,en solution aqueuse ou organique, un agent de chloration tel que le chlore, l'acide hypochloreux, l'acide chlorhydrique en présence d'eau oxygénée,le chlorure de sulfuryle, un N chloroimide comme par exemple le N chloro succinimide ou le pentachlorure de phosphore.

**9)** Procédé selon la revendication 7, caractérisé en ce qu'on fait réagir un agent de bromation tel que le brome en solution aqueuse ou organique, le perbromure de pyridinium.

**10)** Procédé selon la revendication 7, caractérisé en ce qu'on fait agir un agent d'iodation tel que l'iode seule ou en solution acide ou basique.

**11)** Procédé selon la revendication 7, caractérisé en ce qu'on effectue la préparation du tétrafluoroborate du dérivé de diazonium de l'azote en 4 du dérivé de formule I.

**12)** Composition fongicide pour la protection des plantes contre les maladies fongiques, caractérisée en ce qu'elle contient ,comme matière active , un dérivé selon l'une des revendications 1 à 6.

**13)** Utilisation d'un dérivé selon l'une des revendications 1 à 6 pour la protection des plantes contre les maladies fongiques.

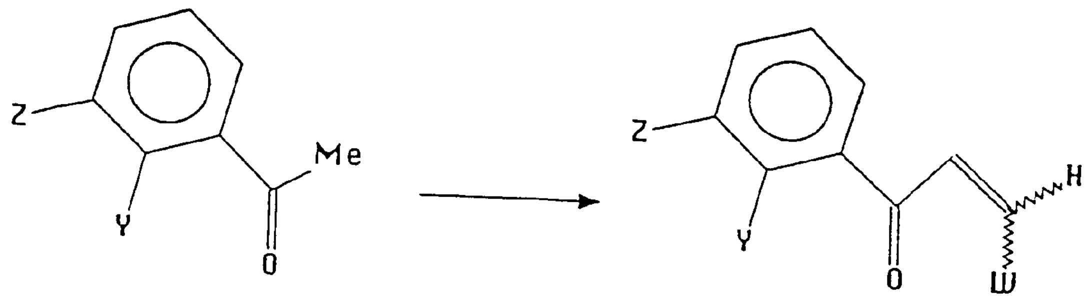

Formule VI

Formule III

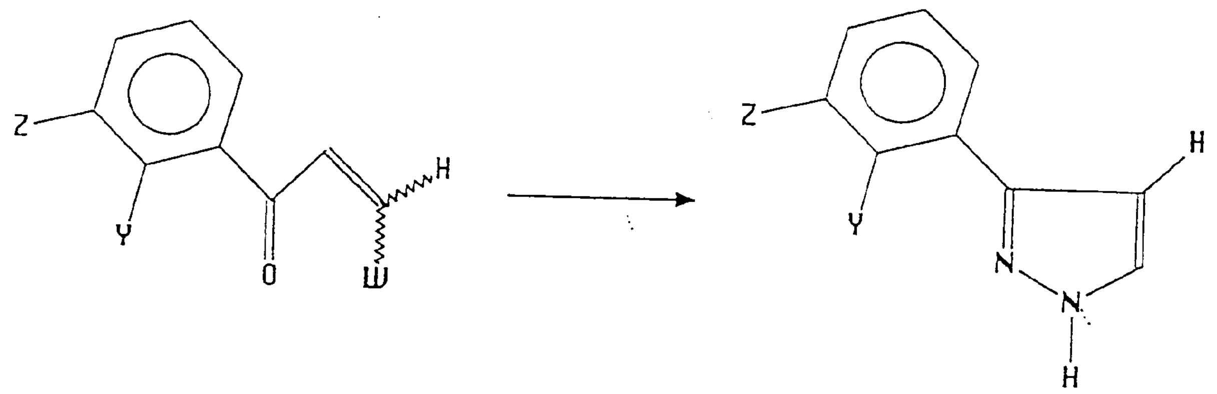

Formule III

Formule II

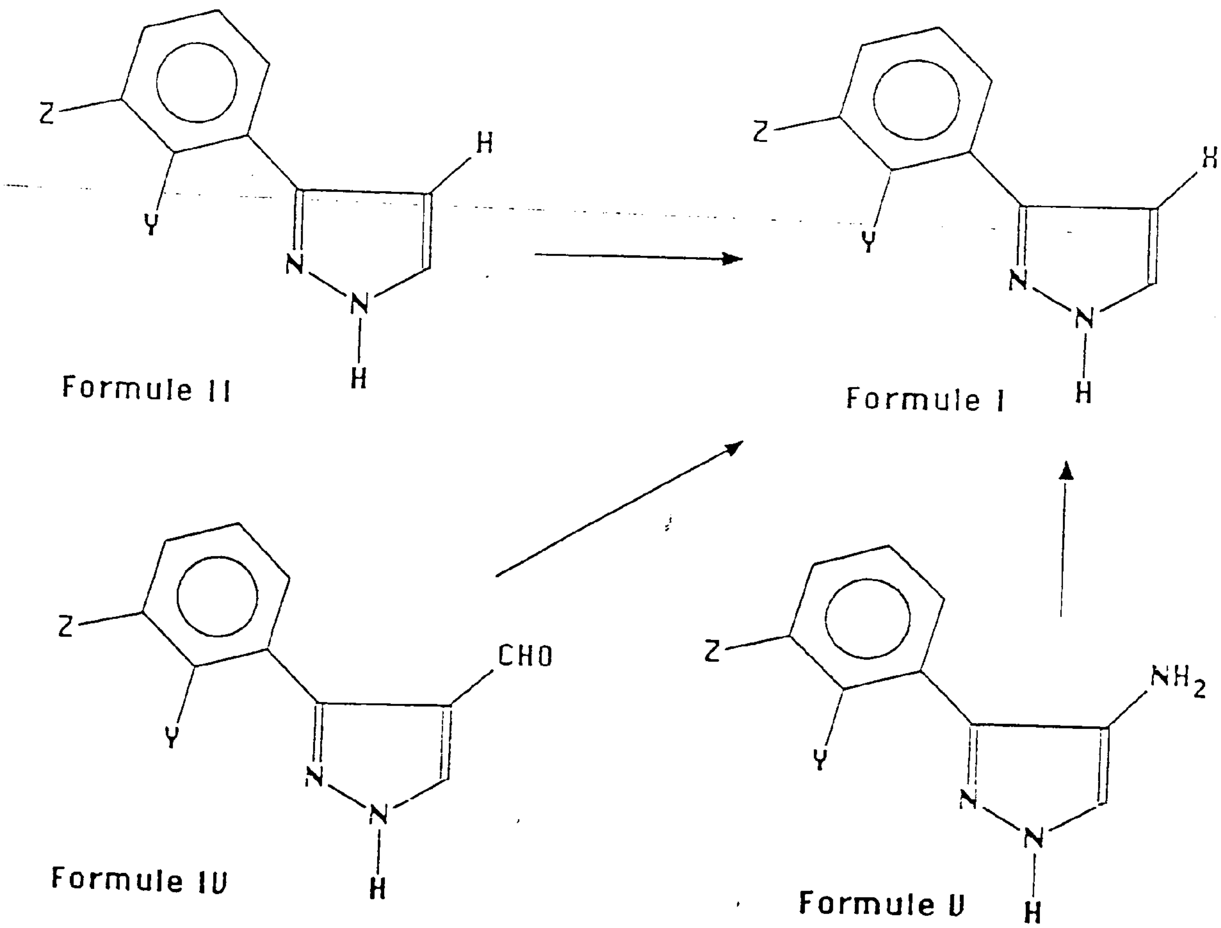

Formule II

Formule I

Formule IV

Formule V

Office européen des brevets

# RAPPORT PARTIEL DE RECHERCHE EUROPEENNE

qui selon la règle 45 de la Convention sur le brevet européen est considéré, aux fins de la procédure ultérieure comme le rapport de la recherche européenne

Numero de la demande

EP 92 42 0359

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 5) |
|---|---|---|---|
| X | US-A-2 721 143 (KNOLL A,G, CHEMISCHE FABRIKEN)<br>* colonne 1, ligne 53 - ligne 63 *<br>----- | 1-13 | C07D231/16<br>A01N43/56<br>C07D231/14<br>C07D231/18<br>C07D405/04<br>C07D401/06<br>C07D401/12<br>C07D405/12<br>C07D409/12<br>C07F9/6506<br>C07F7/08<br>C07D231/12 |

DOMAINES TECHNIQUES RECHERCHES (Int. Cl. 5)

C07D
A01N
C07F

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes:

Revendications ayant fait l'objet de recherches incomplètes:

Revendications n'ayant pas fait l'objet de recherches:

Raison pour la limitation de la recherche:

voir feuille supplémentaire C

| Lieu de la recherche | Date d'achèvement de la recherche | Examinateur |
|---|---|---|
| LA HAYE | 21 DECEMBRE 1992 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04E08)

EP 92 42 0359 -C-

RECHERCHE INCOMPLÈTE

Revendications ayant fait l'objet de recherches incomplètes : 1-13

Raison : La rédaction des revendications n'est pas claire ni concise (Art. 83-84 OEB), et représente une telle masse énorme de produits, qu'une recherche complète n'est pas possible pour des raisons d'économie (voir Directives relatives à l'examen pratiqueé à l'OEB, Partie B, Chapitre III,2). De cette façon la recherche a été fondé sur le concept inventif de la demande telle qu'exemplifié par les composés, qui sont bien caractérisés par des éléments physiques ou chimiques, c.à.d. les composés des exemples.